# EUROPEAN PATENT SPECIFICATION

(11) **EP 0 697 880 B1**
(45) Date of publication and mention of the grant of the patent: **22.08.2001**
(21) Application number: 94914062.8
(22) Date of filing: 06.04.1994
(51) Int. Cl.: A61K 38/02, A61K 39/395

(54) **INHIBITION OF LEUKOCYTE ADHESION**
Inhibierung von Leukozytenadhäsion
INHIBITION DE L'ADHESION DES LEUCOCYTES

(30) Priority: 03.05.1993 US 56454
(43) Date of publication of application: 28.02.1996
(73) Proprietor: GENENTECH, INC., South San Francisco, CA 94080-4990 (US); THE REGENTS OF THE UNIVERSITY OF CALIFORNIA, Oakland, California 94612-3550 (US)
(72) Inventor: LASKY, Laurence, A., Sausalito, CA 94965 (US); BAUMHUETER, Susanne, Redwood City, CA 94061 (US); ROSEN, Steven, D., San Francisco, CA 94703 (US); SINGER, Mark, S., San Francisco, CA 94703 (US)
(74) Representative: Armitage, Ian Michael
(86) International application number: US9403791
(87) International publication number: WO9425047

(56) References cited:
- WO-A-92/19735
- WO-A-93/00919
- JOURNAL OF BIOLOGICAL CHEMISTRY vol. 268, no. 6 , 25 February 1993 , BALTIMORE MD, USA pages 4525 - 4529 D. DOWBENKO ET AL. 'Structure and chromosomal localization of the murine gene encoding GLYCAM 1.'
- THE JOURNAL OF CELL BIOLOGY vol. 114, no. 2 , July 1991 , NEW YORK NY, USA pages 343 - 349 E. BERG ET AL. 'The human peripheral lymph node vascular addressin is a ligand for LECAM-1, the peripheral lymph node homing receptor.' cited in the application
- SCIENCE vol. 262, no. 5132 , 15 October 1993 , WASHINGTON DC, USA pages 436 - 438 S. BAUMHUETER ET AL. 'Binding of L-selectin to the vascular sialomucin CD34.'
- HISTOCHEMISTRY vol. 100, no. 3 , September 1993 pages 185 - 191 S. ROSEN 'L-selectin and its biological ligands.'

## Description

### BACKGROUND OF THE INVENTION

### I. Field of the Invention

This invention relates to the inhibition of intercellular adhesion mediated by L-selectin by administering a newly identified L-selectin ligand, CD34. More particularly, the invention concerns a product for inhibiting leukocyte adhesion to endothelial cells by administering an effective amount of an isolated, purified CD34 polypeptide or an antibody capable of binding native CD34.

### II. Description of Background and Related Art

The migration of leukocytes to sites of acute or chronic inflammation involves adhesive interactions between these cells and the endothelium. This specific adhesion is the initial event in the cascade that is initiated by inflammatory insults, and it is, therefore, of paramount importance to the regulated defense of the organism.

The types of cell adhesion molecules that are involved in the interaction between leukocytes and the endothelium during an inflammatory response currently stands at four: 1. selectins; 2. (carbohydrate and glycoprotein) ligands for selectins; 3. integrins; 4. integrin ligands, which are members of the immunoglobulin gene superfamily.

The selectins are cell adhesion molecules that are unified structurally by the inclusion of lectin, egf-like and complement binding-like domains [Bevilacqua, M.P., *et al.,* Science 243, 1160-1165 (1989); Johnson, *et al.,* Cell 56, 1033-144 (1989); Lasky, L.A., Cell 56, 1045-1055 (1989); Siegelman, M. *et al.,* Science 243, 1165-1172 (1989); Stoolman, L. M., Cell 56; 907-910 (1989)], and functionally by their ability to mediate cell binding through interactions between their lectin domains and cell surface carbohydrate ligands [Brandley, B., *et al.* Cell 63, 861-863 (1990); Springer, T., and Lasky, L. A. Nature 349 196-197 (1991); Bevilacqua, M.P. and Nelson, R.M., J. Clin. Invest. 91, 379-387 (1993)].

There are three members identified so far in the selectin family of cell adhesion molecules: L-selectin (a.k.a. peripheral lymph node homing receptor (pnHR), LEC-CAM-1, LAM-1, gp90^{MEL}, gp100^{MEL}, gp110^{MEL}, MEL-14 antigen, Leu-8 antigen, TQ-1 antigen, DREG antigen), E-selectin (LEC-CAM-2, LECAM-2, ELAM-1) and P-selectin (LEC-CAM-3, LECAM-3, GMP-140, PADGEM).

L-selectin is expressed on the surface of leukocytes, such as lymphocytes, neutrophils, monocytes, and eosinophils, and is involved with the trafficking of lymphocytes to peripheral lymphoid tissues [Gallatin et al., Nature 303, 30-34 (1983)] and with acute neutrophil-mediated inflammatory responses [Watson, S.R., Nature 349 164-167 (1991)]. The amino acid sequence of L-selectin and the encoding nucleic acid sequence are, for example, disclosed in U. S. Patent No. 5,098,833 issued 24 March 1992.

L-selectin, which comprises a lectin domain, performs its adhesive function by recognizing carbohydrate-containing ligands on endothelial cells. Using an L-selectin-IgG chimeric molecule, Imai, Y. *et al*., *J*. Cell Biol. 113 1213-1221 (1991) precipitated an about 50-kD sulfated glycoprotein (Sgp 50) from [³⁵S]sulfate-labeled mesenteric lymph nodes, and proposed that Sgp50 is a HEV ligand for L-selectin. [See also Imai *et al.,* Glycobiology 2, 373-381 (1992); Imai et al., Nature 361, 555-557 (1993); and PCT application publication No. WO 92/19761 published 12 November 1992.] A band of about 90 kD, relatively minor in terms of sulfate incorporation, was also observed in most analyses, and was designated Sgp 90. Among lymphoid tissues, only peripheral lymph nodes (PN) and mesenteric lymph nodes (MLN) showed the 50- and 90-kD bands, while Peyer's patches (PP), spleen, and thymus were found negative for both in the assays used. Nonlymphoid organs such as kidney, liver, cerebrum, and cerebellum were found to be completely negative for both Sgp50 and Sgp90. Sgp 90 shared many features with Sgp 50 and was shown to be a sulfated, fucosylated glycoprotein, the precipitation of which was calcium dependent, inhibitable by the L-selectin specific MEL-14 monoclonal antibody, inhibitable by specific polysaccharides, and dependent on the presence of sialic acid. Similarly to Sgp 50, Sgp 90 was reported to be precipitated by the monoclonal antibody MECA-79 [Streeter *et al.,* Nature (Lond.) 331, 41-46 (1988); Streeter *et al.,* J. Cell. Biol. 107, 1853-1862 (1988)], and it was hypothesized that this component might be identical to the prominent 90-kD component recognized by MECA-79 among several other proteins in a Western blot analysis of lymph node [Butcher *et al.,* Am. J. Pathol. 136, 3-12 (1990)].

Sgp 90 was found to be bound tightly to the endothelial cell surface [Imai et al., J. Cell. Biol. supra; Lasky *et al.,* Cell 69, 927-938 (1992) and Brustein *et al.,* J. Exp. Med. 176, 141501419 (1992)], in contrast to the Sgp 50 ligand that is loosely associated with the endothelial cell. The L-selectin-IgG chimeric molecule was used in combination with physical fractionation and plant lectin chromatography to purify sufficient quantities of the more abundant Sgp 50 ligand for amino acid sequence analysis that enabled the cloning and expression of the cDNA encoding this glycoprotein [Lasky, L. A. *et al.,* Cell 69, 927-938 (1992), and PCT application publication WO92/19735 published 12 November 1992.]

CD34 (also referred to as HPCA-1) is known as a 115-kDa transmembrane glycoprotein (a sialomucin in character) of unknown function that is expressed on human hematopoietic progenitor cells and the small vessel endothelium of a variety of tissues. Its biochemical structure and partial amino acid sequence were described by Watt *et al.,* Leukemia 1, 417 (1987); and Sutherland *et al.,* Leukemia 2, 793 (1988). The gene was mapped to chromosome 1q [Molgaard *et al.,* Leukemia 3, 773 (1989)], and the cloning of cDNA encoding CD34 was reported by Simmons et *al*., J. Immunol. 148, 267-271 (1992); and Brown *et al.,* Int. Immunol. 3, 175-184 (1991).

### SUMMARY OF THE INVENTION

The present invention is in part based on successful purification and characterization of the - 90 kD glycoprotein ligand for L-selectin (Sgp 90). Isolation of Sgp 90 from murine peripheral lymph nodes by a specifically designed multi-step purification procedure, and amino acid sequencing of both the N terminus and an internal tryptic peptide revealed that the polypeptide backbone of this glycoprotein is substantially identical to that of murine CD34. Using a polyclonal antiserum directed against a recombinant form of murine CD34, the present inventors demonstrated that this glycoprotein has an apical distribution on the endothelia of capillaries and peripheral lymph node high endothelial venules (PLN HEV), consistent with a role as an adhesive ligand for L-selectin. This antiserum was further found to recognize Sgp 90 isolated by L-selectin precipitation from lymph nodes. These results together indicate that an endothelial glycoform of CD34 functions as an adhesive ligand for L-selectin. The identification of the second L-selectin ligand as CD34 was highly unexpected in view of the broad distribution of the latter glycoprotein on extra-lymphoid endothelial cells, which were earlier thought not to express ligands for L-selectin.

In one aspect, the present invention relates to:
a) an isolated, purified CD34 polypeptide; or
b) an antibody capable of binding a native CD34 for use in a method of medical treatment, and in particular to the use of these substances for the preparation of a medicament for inhibiting a pathological condition associated with intercellular adhesion mediated by L-selectin.

In specific embodiments, the CD34 polypeptides or anti-CD34 antibodies are used to inhibit lymphocyte adhesion to high endothelial venules (HEV) of primary and secondary lymphoid organs, or neutrophil or monocyte adhesion to vascular endothelium. The administration of CD34 may be combined with the administration of an effective amount of a further therapeutic agent, including, but not limited to, selectins, further selectin ligands, antibodies capable of binding a selectin or a selectin ligand other than CD34, integrins, integrin ligands, antibodies capable of binding an integrin or an integrin ligand, other cell adhesion molecules or their ligands, non-protein (e.g. carbohydrate) antagonists of L-selectin-mediated leukocyte adhesion, antiinflammatory agents, antioxidants, etc.

In all embodiments of the uses herein, the patient is preferably mammal, more preferably human.

In another aspect, the invention concerns medical uses involving targeting a pharmaceutically active agent to endothelial cells, by chemically or physically associating said agent with an antibody capable of binding CD34.

In yet another aspect, the invention concerns a pharmaceutical composition comprising a CD34 polypeptide or an anti-CD34 antibody and optionally a further pharmaceutically active agent, preferably an antiinflammatory agent, an antioxidant or a further inhibitor of leukocyte adhesion to endothelial cells.

In further aspect, the invention concerns a bispecific molecule comprising a CD34 sequence or an antibody sequence capable of binding a native CD34 and a further pharmaceutically active moiety. In a particular embodiment, the bispecific molecule might comprise a first antibody sequence capable of binding a native CD34 and a second antibody sequence capable of binding a different molecule associated with leukocyte adhesion.

In a still further aspect, the invention concerns a molecule comprising a pharmaceutically active moiety covalently linked to an antibody capable of binding a native CD34.

These and other aspects of the invention will be apparent for those skilled in the art.

### BRIEF DESCRIPTION OF THE DRAWINGS

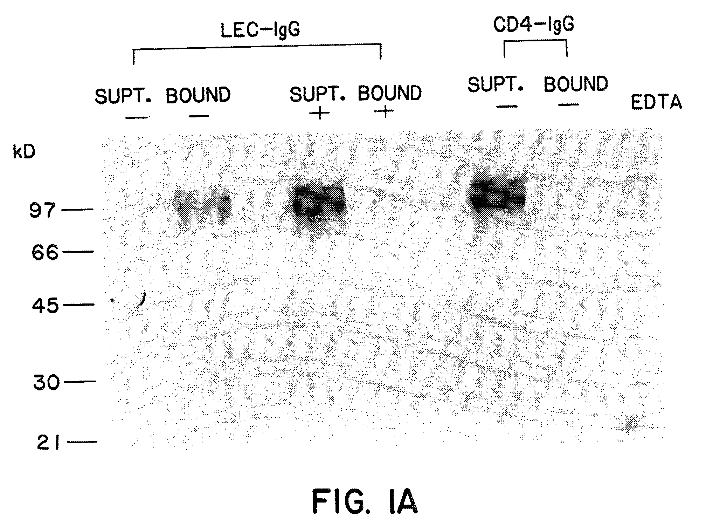

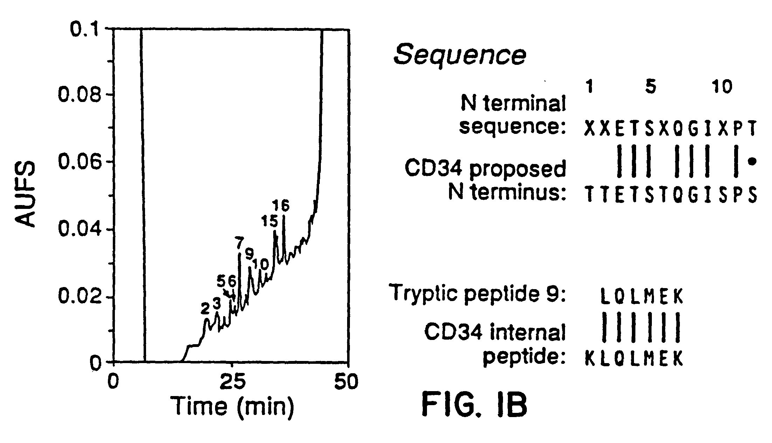

**Figure 1. Purification and amino acid analysis of the Sgp 90 L-selectin ligand. A.** Sgp90 has ligand activity for L-selectin independently of Sgp50. Reprecipitation of SDS-PAGE purified Sgp90 with L selectin-IgG Protein A Sepharose (LEC-IgG) or CD4-IgG Protein A Sepharose (CD4-IgG) in the absence (-) or presence (+) of EDTA. "supt." refers to unbound material in the supernatant, "bound" to precipitated material. Mᵣ standards are shown on the left. B. The N-terminal sequence of the isolated Sgp 90 L-selectin ligand compared with the proposed ¹² N terminus of murine CD34. The "X"s at positions 1,2,6 and 10 refer to gaps in the N-terminal sequence of the Sgp90 ligand that are presumably due to O-glycosylation of these threonine/serine residues. Also shown is the capillary HPLC profile of the tryptic digest of isolated Sgp90 and the sequence of tryptic peptide 9 compared with the sequence of an internal tryptic peptide of murine CD34.

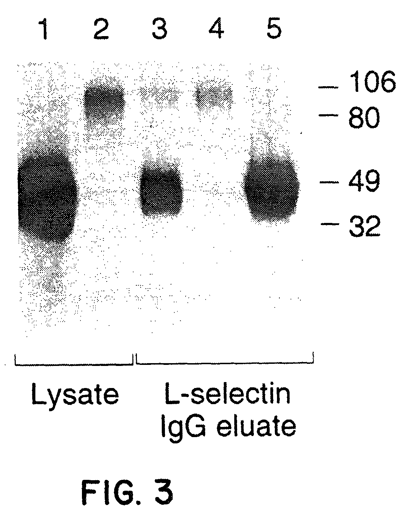

**Figure 3. Immunoprecipitation analysis of sulphate -labeled glycoproteins from murine PLN. 1.** Immunoprecipitation of sulphate labeled PLN lysates with anti-GlyCAM 1 antiserum, **2.** Immunoprecipitation of sulphate labeled PLN lysates with anti-mCD34 antiserum, **3.** Immunoprecipitation of sulphate labeled PLN lysates with L selectin-IgG chimera. The L-selectin-adherent sulphate labeled ligands were released by EDTA incubation and reprecipitated with L-selectin-IgG in the presence of calcium, **4.** Immunoprecipitation of L-selectin adherent, EDTA released sulphate labeled protein with anti-mCD34 antiserum, 5. Immunoprecipitation of L-selectin adherent, EDTA released sulphate labeled protein with anti-GlyCAM 1 antiserum. 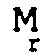 standards are,shown on the right.

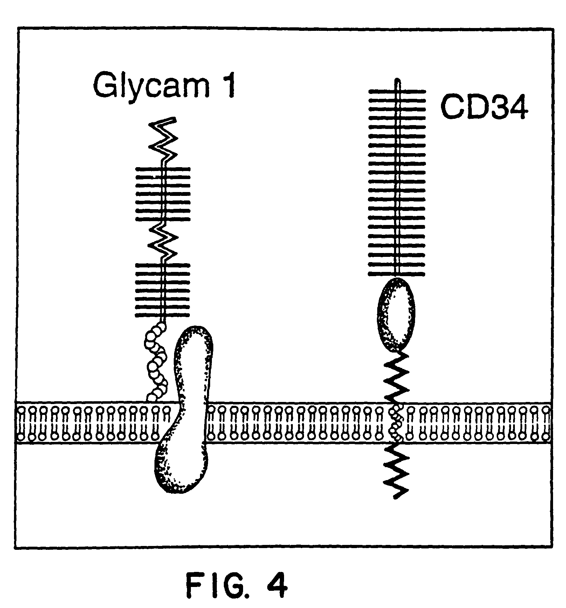

**Figure 4. Comparative hypothetical structures of the sgp50 (GlyCAM 1) and sgp90 (CD34) endothelial ligands for L selectin.** The previously proposed structure of GlyCAM 1 is shown on the left and illustrates the two highly extended mucin-like domains (cross-hatched lines), the potential C-terminal amphipathic helix and a postulated transmembrane protein that interacts with the helical domain. On the right is illustrated a hypothetical structure of mCD34, including a longer highly extended mucin-like domain at the N terminus (cross hatched line), a cysteine rich domain (shaded ellipsoid), a transmembrane domain (helix) and an extended cytoplasmic domain.

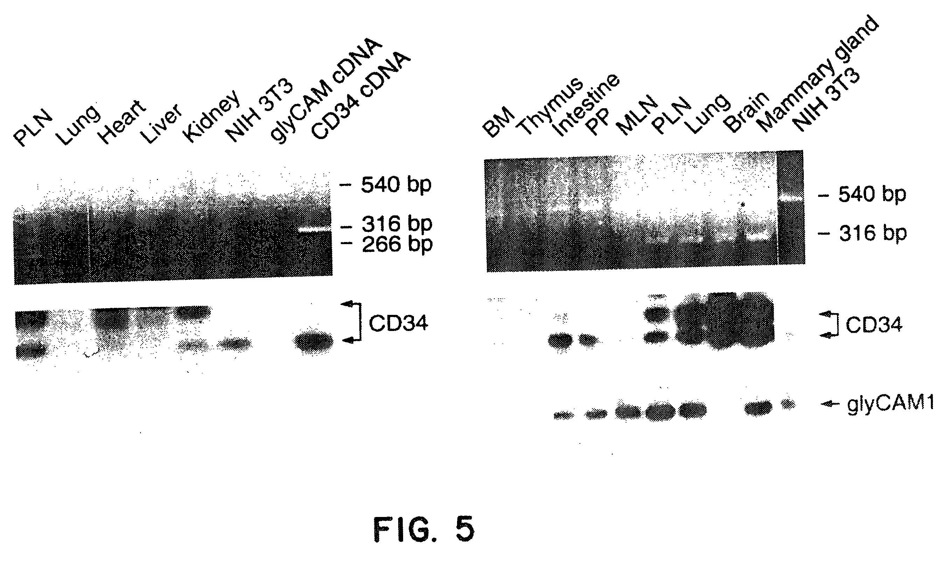

**Figure 5. Expression pattern of mCD34 RNA in various tissues.**

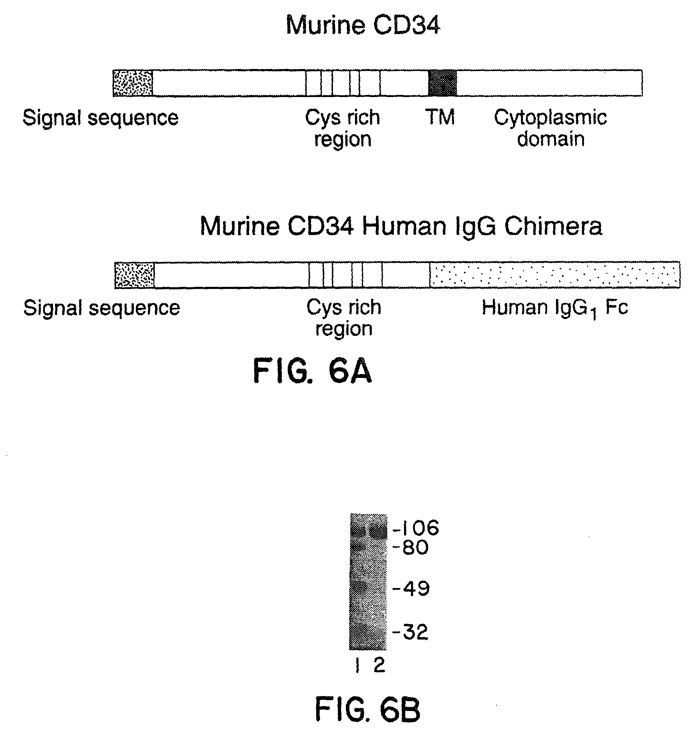

**Figure 6.** (A) Schematic representation of the murine CD34 cDNA (upper) and the mCD34 human IgG chimera (lower), which was stably transfected into 293 cells. (B) Analysis of the affinity purified CD34/lgG fusion protein by reducing SDS polyacrylamide gel electrophoresis; Coomassie blue stained gel is shown with molecular weight markers on the right in kDa.

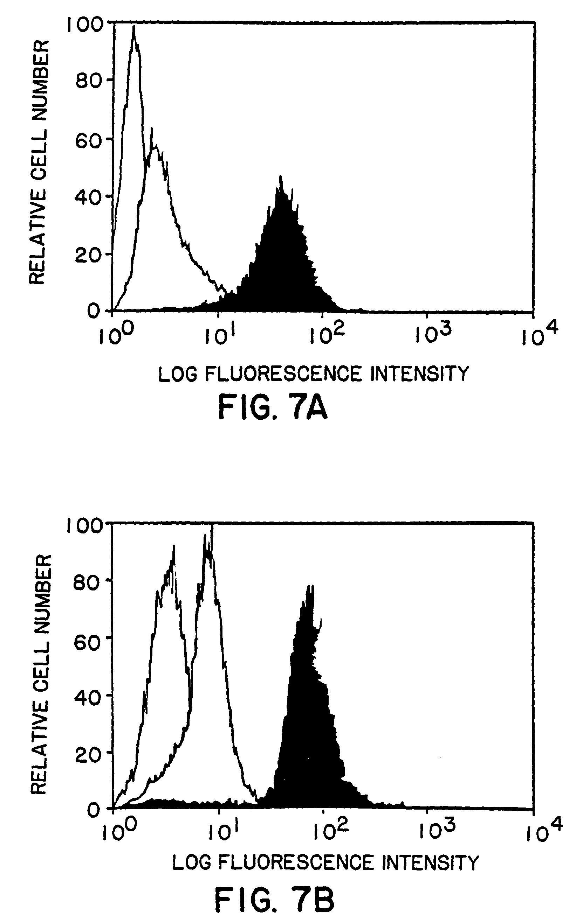

**Figure 7.** Fluorescence activated cell sorter analysis of the surface expression of CD34 on (A) MRK cells transfected with the full length murine CD34 cDNA and (B) NIH 3T3 cells that were shown to express high levels of mRNA. Profiles shown are cells stained with the secondary antibody only (no fill), with preimmune serum (gray fill) and with anti CD34 antiserum (black fill).

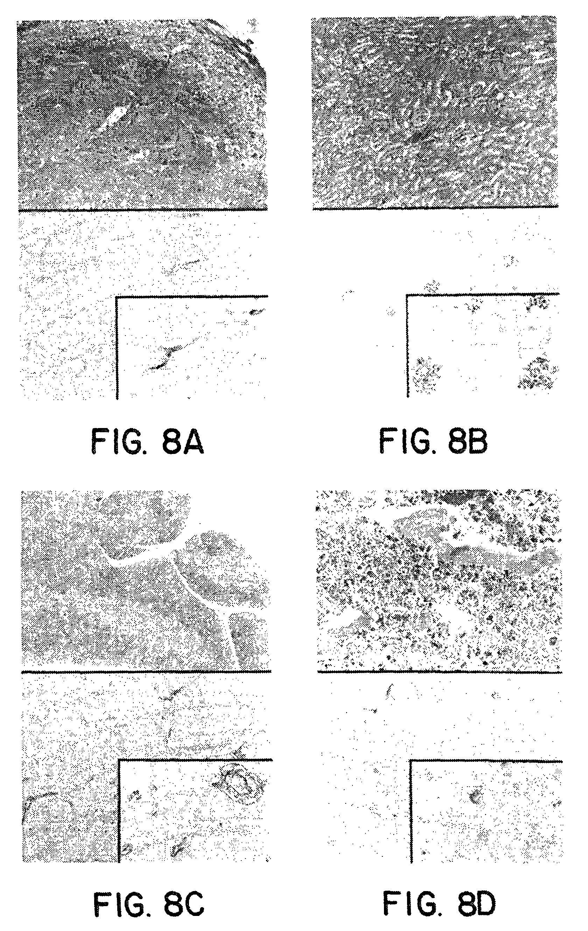

**Figure 8.** Immunohistochemical analysis of the vascular distribution of CD34 using the anti CD34 antibody. Sections were prepared and stained as detailed in Materials and Methods. (A) brain, (B) kidney, (C) thymus and (D) bone marrow. The upper panels are hematoxylin/eosin stained sections shown at 10x original magnification, the lower panels are stained with anti CD34 (10x) and the insets in the right hand corner represent a detail at 40x original magnification.

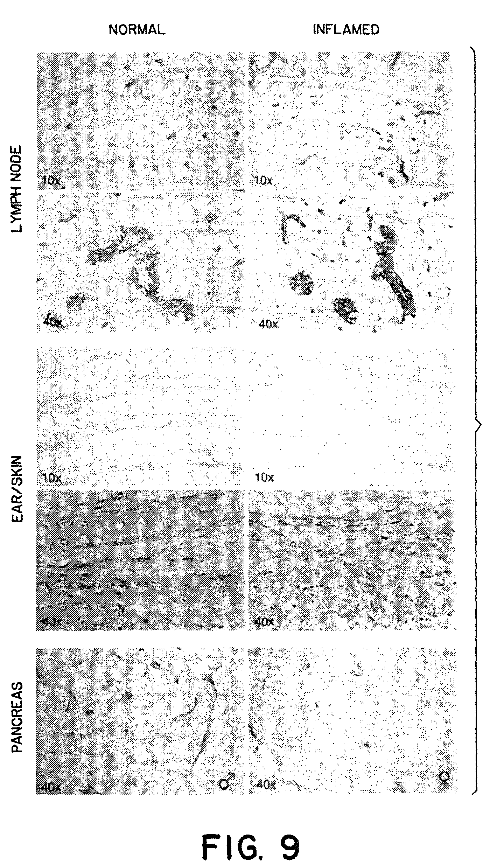

**Figure 9.** Regulation of vascular CD34 expression in inflamed tissues. (A) normal and inflamed lymph node sections, (B) normal skin or skin from animals after induction of a DTH response, (C) pancreatic islets from male or female NOD mice at 20 weeks of age stained as described in Materials and Methods. HEV like structures expressing CD34 in the female NOD pancreas are indicated by arrowheads.

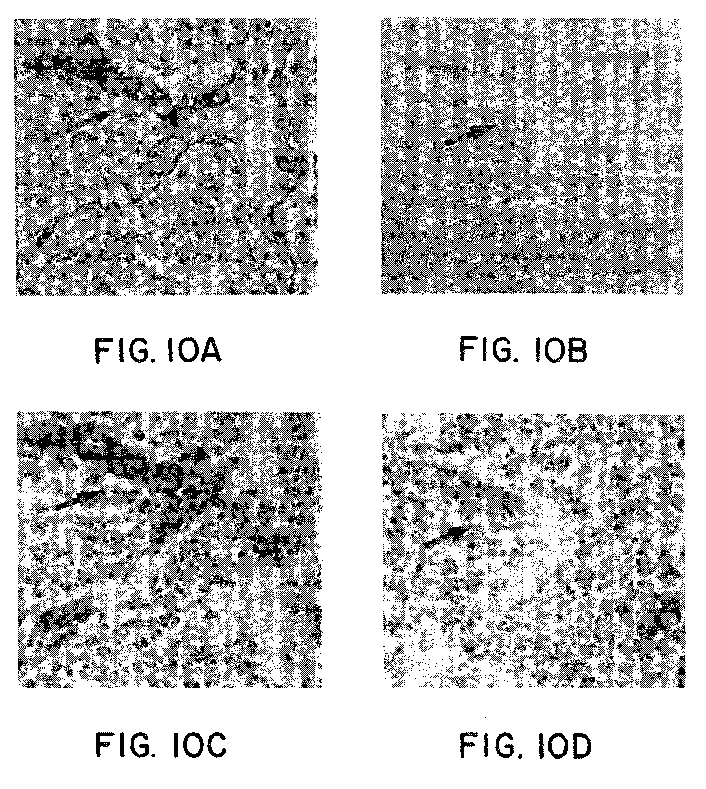

**Figure 10.** L-selectin/IgG binding to HEV like structures in the inflammatory infiltrate of pancreatic islets. Colocalization with GlyCAM 1 and CD34. Serial sections of fresh frozen tissue were stained with (A) immunogold labeled L-selectin/IgG, (B)immunogold labeled L-selectin/IgG in the presence of EGTA, (C) anti GlyCAM 1 and (D) anti CD34 as detailed in Materials and Methods. HEV like structures are clearly stained (see arrowheads). The anti CD34 antibody only weakly reacts when fresh frozen tissue sections are used which are required for binding of L-selectin/IgC.

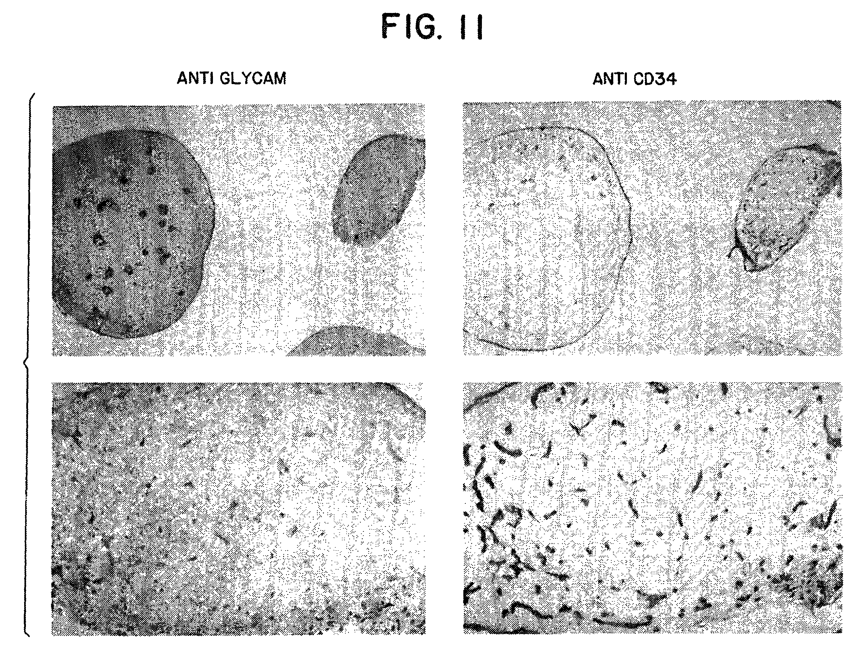

**Figure 11.** CD34 expression in normal versus deafferentiated peripheral lymph nodes. (A) Detection of GlyCAM 1 in normal but not deafferentiated lymph nodes (arrow and detail in lower panel), (B) unchanged expression of CD34 in deafferentiated lymph nodes (arrow and detail in lower panel).

### DETAILED DESCRIPTION OF THE INVENTION

### I. Definitions

The term "CD34 polypeptide" is used to refer to native CD34 molecules and their derivatives, either occurring in nature or produced by chemical synthesis or recombinant DNA technology, provided that they retain the qualitative ability to bind L-selectin. Ordinarily a CD34 polypeptide as defined herein shall have a mucin-like domain at the N terminus, followed by a cysteine rich domain, a transmembrane domain and a cytoplasmic domain, nonetheless may contain fewer domains or may have some of the domains repeated provided that the ability to bind L-selectin is retained.

The terms "native CD34" and "wild-type CD34" are used interchangeably and refer to a CD34 molecule as occurring in nature ("native sequence CD34") in any animal species, including humans, either purified from natural. source, chemically synthesized or recombinantly produced. It will be understood that natural allelic variations exist and can occur among individuals, as demonstrated by one or more amino acid differences in the amino acid sequence of each individual. These allelic variations are specifically within the scope of native CD34.

The term "isolated, purified CD34 polypeptide" is used to refer to a CD34 polypeptide as hereinabove defined, which is substantially devoid of other proteins, gives a single lane on an SDS 10% polyacrylamide gel, and is in isolated form. "Isolated, purified native CD34" is substantially unaccompanied by other polypeptides with which it is ordinarily associated in its native environment.

Antibodies (Abs) and immunoglobulins (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one and (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains [Clothia *et al.,* J. Mol. Biol. 186, 651-663 (1985); Novotny and Haber, Proc. Natl. Acad. Sci. USA 82, 4592-4596 (1985)].

The variability is not evenly distributed through the variable regions of antibodies. It is concentrated in three segments called complementarity determining regions (CDRs) or hypervariable regions both in the light chain and the heavy chain variable regions. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies [see Kabat, E.A. *et al.,* Sequences of Proteins of Immunological Interest National Institute of Health, Bethesda, MD (1987)]. The constant domains are not involved directly in binding an antibody to an antigen, but exhibit various effector functions, such as participation of the antibody in antibody-dependent cellular toxicity.

Papain digestion of antibodies produces two identical antigen binding fragments, called Fab fragments, each with a single antigen binding site, and a residual "Fc" fragment, whose name reflects its ability to crystallize readily. Pepsin treatment yields an F(ab')₂ fragment that has two antigen combining sites and is still capable of cross-linking antigen.

"Fv" is the minimum antibody fragment which contains a complete antigen recognition and binding site. This region consists of a dimer of one heavy and one light chain variable domain in tight, non-covalent association. It is in this configuration that the three CDRs of each variable domain interact to define an antigen binding site on the surface of the V_{H}-V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (C_{H}1) of the heavy chain. Fab' fragments differ from Fab fragments by the addition of a few residues at the carboxy terminus of the heavy chain C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other, chemical couplings of antibody fragments are also known.

The light chains of antibodies (immunoglobulins) from any vertebrate species can be assigned to one of two clearly distinct types, called kappa and lambda (λ), based on the amino acid sequences of their constant domains.

Depending on the amino acid sequence of the constant region of their heavy chains, immunoglobulins can be assigned to different classes. There are five major classes of immunoglobulins: IgA, IgD, IgE, IgG and IgM, and several of these may be further divided into subclasses (isotypes), e.g. IgG-1, IgG-2, IgG-3, and IgG-4. The heavy chain constant regions that correspond to the different classes of immunoglobulins are called α, delta, epsilon, γ, and µ, respectively. The subunit structures and three-dimensional configurations of different classes of immunoglobulins are well known.

The term "antibody" is used herein in the broadest sense and specifically covers single monoclonal antibodies, immunoglobulin chains or fragments thereof, which react immunologically with native CD34 molecules, as well as antibody compositions with polyepitopic specificity, which have such properties.

The term "monoclonal antibody" as used herein refers to an antibody (as hereinabove defined) obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins.

"Humanized" forms of non-human (e.g. murine) antibodies are immunoglobulins, immunoglobulin chains or fragments thereof (such as Fv) Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues which are found neither in the recipient antibody nor in the imported CDR or framework sequences. These modifications are made to further refine and optimize antibody performance.

The monoclonal antibodies herein include hybrid (chimeric) and recombinant antibodies produced by splicing a variable (including hypervariable) domain of an antibody with a constant domain (e.g. "humanized" antibodies), only one of which is directed against the indicated polypeptide, or a light chain with a heavy chain, or a chain from one species with a chain from another species, or fusions with heterologous proteins, regardless of species of origin or immunoglobulin class or subclass designation, as well as antibody fragments (e.g., Fab, F(ab')₂, and Fv). [See, e.g. Cabilly, et al., U.S. Pat. No. 4,816,567; Mage & Lamoyi, in Monoclonal Antibody Production Techniques and Applications, pp.79-97 (Marcel Dekker, Inc., New York, 1987).]

For "chimeric" and "humanized" antibodies see, for example, U.S. Patent No. 4,816,567; WO 91/09968; EP 452,508; and WO 91/16927).

Thus, the modifier "monoclonal" indicates the character of the antibody as being obtained from a substantially homogeneous population of antibodies, and is not to be construed as requiring production of the antibody by any particular method.

The antibodies herein may be of any immunoglobulin class or isotype, such as IgG-1, IgG-2, IgG-3, or IgG-4 subtypes, IgA, IgE, IgD or IgM. If an immunoglobulin effector function is desirable, the antibodies herein typically retain at least functionally active hinge, CH2 and CH3 domains of the constant region of the heavy chain of an immunoglobulin.

As used herein the phrase "bispecific antibody" designates antibodies (as hereinabove defined) having at least two binding specificities. Bispecific antibodies can generally be assembled as hetero-multimers, and particularly as hetero-dimers, -trimers or -tetramers, essentially as disclosed in WO 89/02922 (published 6 April 1989), in EP 314,317 (published 3 May 1989), and in U.S. Patent No. 5,116,964 issued 2 May 1992.

In general, the anti-CD34 antibodies used in accordance with the present invention include derivatives of native-sequence antibodies.

The term "derivative" is used to define amino acid sequence and glycosylation variants, and covalent modifications of a native CD34 molecule or an antibody capable of binding a native CD34 molecule (anti-CD34 antibody).

The terms "amino acid" and "amino acids" refer to all naturally occurring L-α-amino acids. The amino acids are identified by either the single-letter or three-letter designations:

| | | | | | |
|---|---|---|---|---|---|
| Asp | D | aspartic acid | Ile | I | isoleucine |
| Thr | T | threonine | Leu | L | leucine |
| Ser | S | serine | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |
| Val | V | valine | Gln | Q | glutamine |
| Met | M | methionine | Asn | N | asparagine |

These amino acids may be classified according to the chemical composition and properties of their side chains. They are broadly classified into two groups, charged and uncharged. Each of these groups is divided into subgroups to classify the amino acids more accurately:

### I. Charged Amino Acids

Acidic Residues: aspartic acid, glutamic acid
Basic Residues: lysine, arginine, histidine

### II. Uncharged Amino Acids

Hydrophilic Residues: serine, threonine, asparagine, glutamine
Aliphatic Residues: glycine, alanine, valine, leucine, isoleucine
Non-polar Residues: cysteine, methionine, proline
Aromatic Residues: phenylalanine, tyrosine, tryptophan

The term "amino acid sequence variant" refers to molecules with some differences in their amino acid sequences as compared to a native sequence CD34 molecule or antibody, including molecules comprising substitutions, insertions and/or deletions as compared to a native CD34 or anti-CD34 antibody amino acid sequence. The term specifically includes fragments, i.e. polypeptide subsets of native sequence molecules and substitution, insertion and/or deletion variants of such fragments. Ordinarily, the amino acid sequence variants will possess at least about 40% homology, more preferably at least about 60% homology, still more preferably 70% homology, even more preferably at least about 80% homology, and most preferably at least about 90% homology with the amino acid sequence of the corresponding native CD34 or antibody. The degree of homology is preferably highest in regions directly participating in L-selectin binding, including but not limited to the highly O-glycosylated mucin-like domain of CD34, which is believed to function as a scaffold for the polyvalent presentation of the appropriate, sulphated and sialylated carbohydrate structure to the L-selectin lectin domain.

"Homology" is defined as the percentage of residues in the candidate amino acid sequence that are identical with the residues in the amino acid sequence of a native molecule after aligning the sequences and introducing gaps, if necessary, to achieve the maximum percent homology. Methods and computer programs for the alignment are well known in the art.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule. Substantially changes in the properties of a native CD34 ligand may be obtained by substituting an amino acid with a side chain that is significantly different in charge and/or structure from that of the native amino acid. This type of substitution would be expected to affect the structure of the polypeptide backbone and/or the chare or hydrophobicity of the molecule in the area of substitution.

Moderate changes in the ligand properties would be expected by substituting an amino acid with a side chain that is similar in charge and/or structure to that of the native molecule. This type of substitution, referred to as conservative substitution, would not be expected to substantially alter either the structure of the polypeptide backbone or the charge or hydrophobicity of the molecule.

Insertional variants are those with one or more amino acids inserted immediately adjacent to an amino acid at a particular position in a native CD34 sequence. Immediately adjacent to an amino acid means connected to either the α-carboxy or α-amino functional group of the amino acid. Ordinarily, the insertion will consist of one or two conservative amino acids. Amino acids similar in charge and/or structure to the amino acids adjacent to the site of insertion are defined as conservative. Alternatively, this invention includes insertion of an amino acid with a charge and/or structure that is substantially different from the amino acids adjacent to the site of insertion. Such variants may, for example, be prepared with the aim of increasing binding affinity to L-selectin.

Deletional variants are those with one or more amino acids in the native CD34 amino acid sequence removed. Deletional variants, as defined for the purpose of the present invention, include derivatives of native CD34 molecules from which one or more domains have been entirely deleted.

Of particular interest are soluble amino acid sequence variants of CD34. Such soluble CD34 molecules are not bound to the cell membrane due to the deletion or inactivation of their transmembrane domain.

The term "glycosylation variant" is used to refer to a CD34 polypeptide or anti-CD34 antibody having a glycosylation profile different from that of a native CD34. Glycosylation of polypeptides is typically either N-linked or O-linked. N-linked refers to the attachment of the carbohydrate moiety to the side-chain of an asparagine residue. The tripeptide sequences, asparagine-X-serine and asparagine-X-threonine, wherein X is any amino acid except proline, are recognition sequences for enzymatic attachment of the carbohydrate moiety to the asparagine side chain. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be involved in O-linked glycosylation. The CD34 polypeptides of the present invention are characterized by the predominance of O-linked glycosylation, although CD34 on human progenitor cells (i.e. stem cells) has been shown to have both N-linked and O-linked carbohydrate chains [Sutherland, D.R. *et al*., Leukemia 2, 793-803 (1988)]. Lymph node CD34 was found to be sulfated, fucosylated, sialylated and being cross-reactive with the carbohydrate-directed MECA-79 monoclonal antibody. As the interaction of CD34 with the lectin domain of L-selectin is carbohydrate-mediated, any alteration in the glycosylation pattern is expected to result in molecules the receptor binding activities of which will be significantly different from that of the corresponding native CD34 ligand. Any difference in the location and/or nature of the carbohydrate moieties present in a ligand as compared to its native counterpart is within the scope herein, provided that the resultant glycoprotein retains the qualitative ability to bind L-selectin. Various CD34 polypeptides containing distinct patterns of glycosylation may be able to selectively inhibit L-selectin mediated adhesion to endothelial cells at different locations of the body.

The glycosylation pattern of native ligands can be determined by known techniques of analytical chemistry, including HPAE chromatography [Hardy, M.R. *et al.,* Anal. Biochem. 170, 54-62 (1988)], methylation analysis to determine glycosyl-linkage composition [Lindberg, B., Meth. Enzymol. 28. 178-195 (1972); Waeghe, T.J. *et al.,* Carbohydr. Res. 123, 281-304 (1983)], NMR spectroscopy, mass spectrometry, etc.

Covalent derivatives are characterized by containing additional chemical moieties that are not ordinarily parts of the native CD34 molecule or antibody. "Covalent derivatives" include modifications of a native CD34 molecule or anti-CD34 antibody with an organic proteinaceous or nonproteinaceous derivatizing agent, and post-translational modifications. Covalent modifications are traditionally introduced by reacting targeted amino acid residues of the ligand with an organic derivatizing agent that is capable of reacting with selected side-chains or terminal residues, or by harnessing mechanisms of post-translational modifications that function in selected recombinant host cells. Such modifications are within the ordinary skill in the art and are performed without undue experimentation. Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions. Either form of these residues may be present in the CD34 ligands used in accordance with the present invention. Other post-translational modifications include hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)].

Covalent derivatives specifically include fusion molecules in which a native CD34 or anti-CD34 antibody, or their amino acid sequence or glycosylation variants are covalently bonded to a nonproteinaceous polymer. The nonproteinaceous polymer ordinarily is a hydrophilic synthetic polymer, i.e. a polymer not otherwise found in nature. However, polymers which exist in nature and are produced by recombinant *or in vitro* methods are useful, as are polymers which are isolated from nature. Hydrophilic polyvinyl polymers fall within the scope of this invention, e.g. polyvinylalcohol and polyvinylpyrrolidone. Particularly useful are polyvinylalkylene ethers such a polyethylene glycol, polypropylene glycol.

The CD34 or anti-CD34 antibodies may be linked to various nonproteinaceous polymers, such as polyethylene glycol, polypropylene glycol or polyoxyalkylenes, in the manner set forth in U.S. Patent Nos. 4,640,835; 4,496,689; 4,301,144; 4,670,417; 4,791,192 or 4,179,337.

In an another embodiment, the covalent derivatives of CD34 include fusions to stable plasma proteins. "Stable plasma proteins" are proteins typically having about 30 to about 2000 residues, which exhibit in their native environment an extended half-life in the circulation, i.e. a half-life greater than about 20 hours. Examples of suitable stable plasma proteins are immunoglobulins, albumin, lipoproteins, apolipoproteins and transferrin. The CD34 polypeptide amino acid sequence is generally fused C-terminally to a stable plasma protein sequence, e.g. human serum albumin sequence or immunoglobulin constant domain sequence.

A further preferred group of covalent derivatives, a CD34 polypeptide or an anti-CD34 antibody is fused to a toxin moiety. The binding of such fusion molecule (also referred to as chimera or conjugate) to a cell brings the toxin moiety into close proximity with the cell and thereby promotes cell death. Any suitable toxin moiety may be employed; however, it is preferable to employ toxins such as, for example, ricin toxin, diphtheria toxin, membrane channel-forming toxins, radioisotopic toxins, etc.

The term "effective amount" is used to refer to an amount effective in blocking the binding of a native L-selectin receptor to its endothelial ligand.

The term "therapeutically effective amount" is used to refer to an amount sufficient to prevent or suppress the indicated physiological condition or symptom, e.g. inflammation.

### II. GENERAL METHODS

As it should be apparent from the specific Example hereinafter, the purification of the native 90 kD L-selectin ligand (Sgp 90) from mouse lymph nodes, which was subsequently identified as essentially having the amino acid sequence of CD34, was not without difficulties. Sgp 90 is present in much smaller quantities than Sgp 50 and is bound tightly to the endothelial cell surface, while Sgp 50 is loosely associated with the endothelial cells. Accordingly, in contrast to the mouse Sgp 50 ligand (designated mouse GIyCAM-1 in its purified form), which could be purified from conditioned medium by chloroform:methanolextraction, the purification scheme of Sgp 90 had to be specifically designed to employ a detergent for extraction and to include a boiling step, followed by affinity chromatography on wheat germ agglutinin and on an L-selectin-IgG affinity column. The addition of ³⁵S-sulphate labeled PLN extract enabled monitoring of the purification. The final EDTA-released fraction from the L-selectin-IgG affinity column was electrophoresed on an SDS-polyacrylamide gel, transferred to ProBlott membranes, and the Sgp 90 ligand was localized by autoradiography. This purification scheme enabled the isolation of the 90 kD L-selectin ligand in quantities sufficient for amino acid sequence analysis and for cloning the cDNA encoding this glycoprotein. The specific method for isolating the native CD34 polypeptide from mouse lymph nodes is disclosed in the Example hereinafter. CD34 from other animal species (including humans) may be isolated in an analogous manner.

A major achievement of the present invention is, however, that as a result of determining the nucleotide and amino acid sequence of the 90 kD L-selectin ligand, it is no longer necessary to obtain this glycoprotein from its native source by laborious purification procedures. It is now possible to obtain native sequence L-selectin ligand and L-selectin ligand derivatives by expressing the encoding nucleotide sequence in a suitable host cell, including mammalian cells and microorganisms.

General methods applicable for obtaining DNA encoding a CD34 polypeptide, and for the construction of native CD34 and derivatives, including amino acid sequence and glycosylation variants and covalent derivatives, are known in the art and are, for example, disclosed in PCT application publication No. WO 92/19735 published 12 November 1992.

Antibodies capable of binding a native CD34 molecule may be produced by any method known in the art. The following is a brief discussion of certain commonly used techniques that can be used for making such antibodies. Further details of these and similar techniques are found in general textbooks, such as, for example, Cabilly, *et al*., U. S. Patent No. 4,816,567; Mage & Lamoyi, supra; Sambrook *et al.,* Molecular Cloning: A laboratory Manual, Second Edition, Cold Spring Harbor Laboratory Press, New York, 1989; and Current Protocols in Molecular Biology, Ausubel *et al.* eds., Green Publishing Associates and Wiley-Interscience, 1991.

For example, the monoclonal antibodies to be used in accordance with the present invention may be made by the hybridoma method first described by Kohler & Milstein, Nature 256:495 (1975), or may be made by recombinant DNA methods [Cabilly, et al., supra].

In the hybridoma method, a mouse or other appropriate host animal, such as hamster is immunized with a human CD34 polypeptide. Since CD34 is naturally expressed on the surface of various lymphoid and extra-lymphoid endothelial cells, the introduction of such cells into an appropriate animal by subcutaneous, intraperitoneal, or intramuscular routes, will elicit lymphocytes that produce or are capable of producing antibodies that will specifically bind to the CD34 protein used for immunization. Alternatively, lymphocytes may be immunized in vitro. Lymphocytes are then fused with myeloma cells using a suitable fusing agent, such as polyethylene glycol, to form a hybridoma cell [Goding, J. Monoclonal Antibodies: Principles and Practice, pp.59-103 (Academic Press, 1986)].

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high level expression of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 cells available from the American Type Culture Collection, Rockville, Maryland USA. Human myeloma and mouse-human heteromyeloma cell lines also have been described for the production of human monoclonal antibodies. Kozbor, J. Immunol. 133:3001 (1984). Brodeur, et al., Monoclonal Antibody Production Techniques and Applications, pp.51-63 (Marcel Dekker, Inc., New York, 1987).

Culture medium in which hybridoma cells are growing is assayed for production of monoclonal antibodies directed against CD34. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The monoclonal antibodies for use in the method and compositions of the invention are those that preferentially immunoprecipitate CD34 that is present in a test sample, or that preferentially bind to CD34 receptor in a binding assay, and are capable of blocking the adhesion of L-selectin bearing lymphocytes to CD34-bearing endothelial cells in an *in vitro* or *in vivo* cell adhesion assay.

After hybridoma cells are identified that produce antibodies of the desired specificity, affinity, and activity, the clones may be subcloned by limiting dilution procedures and grown by standard methods (Goding, J., Supra). Suitable culture media for this purpose include, for example, Dulbecco's Modified Eagle's Medium or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography. Methods for purification of monoclonal antibodies are well known in the art, and are, for example disclosed in Unit 11.11 of "Current Protocols in Molecular Biology", supra, and in the references cited therein.

The amount of a specific antibody present in a hybridoma supernatant can be quantitated by either solid-phase radioimmunoassay (RIA) or by direct enzyme-linked immunoabsorbent assay (ELISA). In the solid-phase radioimmunoassay, serially diluted antiserum is incubated in microtiter wells previously coated with CD34. Bound antibody is detected by employing ¹²⁵I-labeled anti-immunoglobulin antibodies. The amount of the specific antibody in the antiserum is then determined from a standard curve generated with a specific antibody of known concentration. The unknown antiserum and the standard antibody are assayed in parallel. Protocols for the RIA procedure as used for isotype determination, and the ELISA procedure are, for example, available from Section V of "Current Protocols in Molecular Biology", supra, and from the references cited therein.

DNA encoding the monoclonal antibodies useful in the method of the invention is readily isolated and sequenced using conventional procedures (e.g., by using oligonucleotide probes that are capable of binding specifically to genes encoding the heavy and light chains of murine antibodies). The hybridoma cells described hereinabove serve as a preferred source of such DNA. Once isolated, the DNA may be placed into expression vectors, which are then transfected into host cells such as simian COS cells, Chinese Hamster ovary (CHO) cells, or myeloma cells that do not otherwise produce immunoglobulin protein, to obtain the synthesis of monoclonal antibodies in the recombinant host cells.

### III. ASSAYS

The ability of a particular purified CD34 polypeptide or anti-CD34 antibody to inhibit leukocyte adhesion to endothelial cells can be tested in standard *in vitro* adherence assays. A particularly suitable assay is a modified version of the basic Stamper-Woodruff *in vitro* adherence assay [Stamper and Woodruff, J. Exp. Med. 144, 828-833 (1976)], as disclosed by True *et al.,* J. Cell. Biology 111, 2757-2764 (1990). Without exception in wide variety of studies, this assay has provided a reliable index of the ability of lymphocytes to interact with HEV *in vivo.* For further details see, Rosen, D.S., Current Opinion in Cell Biol. 1, 913-919 (1989).

Alternatively, or in addition, the ability of a CD34 polypeptide or anti-CD34 antibody to inhibit intracellular adhesion mediated by L-selectin can be tested by the assay disclosed in PCT application Publication No. WO 92/19761 published 12 November 1992. According to this method, which is particularly suitable for screening antibodies, the test compound is contacted with L-selectin and an isolated L-selectin-binding agent, which might be a compound comprising the extracellular region of a native CD34 molecule, and its ability to inhibit binding between L-selectin and the isolated L-selectin binding agent is detected. L-selectin may be present as a fusion protein (chimera) comprising an immunoglobulin sequence, preferably an immunoglobulin constant domain (e.g. L-selectin-immunoglobulin chimera, also referred to an immunoadhesin), and/or may be immobilized on a solid surface. The binding between L-selectin and the L-selectin binding agent may, for example, be detected by flow cytometry. Suitable *in vivo* assays might be based on blocking the *in vivo* homing of lymphocytes to lymph nodes, such as, for example, described by Gallatin, W.M., Nature 304, 30-34 (1983).

In addition, the ability of a CD34 polypeptide or anti-CD34 antibody to inhibit L-selectin-mediated pathological conditions, such as inflammation, can be tested in known animal disease models. For example, the ability of a CD34 polypeptide or anti-CD34 antibody to control inflammatory bowel disease (IBD) can be tested in known animal models of IBD. The first group of animal models of IBD includes animals spontaneously developing diseases reminiscent of some forms of IBD. Spontaneous animal models include C3H/HeJ mouse, Japanese waltzing mice, swine dysentry and equine colitis, caused by C. difficile, and the cotton top tamarin. The diseases that these animals suffer have recently been subdivided into five types, two of which resemble UC. Of these models, tamarin are preferred, as a large proportion of these animals have some form of gut disorder, and many of them also develop bowel cancer, as do patients with UC.

In another approach, various irritants, such as ethanol, acetic acid, formalin, immune complexes, trinitrobenzene sulphonic acid (TNBS), bacterial products or carrageenan are used to generate acute or chronic inflammation. A model of this kind has been developed by Wallace, J. and coworkers [Morris *et al.* Gastroenterology 96, 795 (1989)].

According to a third approach, transgenic animals are used to model IBD. Most human patients who have ankylosing spondylitis also carry the gene for HLA-B27. It has been observed that such patients are at greater risk of developing IBD. HLA-B27 transgenic rats, which were attempted to model spondyloarthropathies, in addition to the joint disease, also showed symptoms of chronic inflammation of the bowel which, though not identical, had many similarities with CD. Accordingly, the HL-B27 transgenic rats can be used to model IBD.

Usually the screening for CD34 polypeptides and anti-CD34 antibodies suitable for the purpose of the present invention includes a combination of various *in vitro* and *in vivo* assays.

### III. THERAPEUTIC APPLICATIONS

The methods of the present invention concern the treatment (including prevention) of pathological conditions associated with L-selectin mediated cell adhesion, including autoimmune and inflammatory responses.

A remarkable feature of the immune system is the ability of its B and T cells to recognize almost all foreign molecules. Immune responses are initiated in the widely distributed and numerous peripheral lymphoid organs that guard the body's portals of entry for antigens. The organs that drain most interstitial spaces are the lymph nodes, the spleen monitors the blood, and gut-associated lymphoid aggregates such as Peyer's patches protect against antigens escaping from the gut. Whereas the continuous flux of lymphocytes from one lymphoid organ to the next with the lymphocytes alternatively traveling via the blood and the lymphatics is essential for normal immune response, lymphocytes may also be mediators of pathologic responses, such as pathologic tissue damage as occurs in psoriasis, rheumatoid arthritis and other autoimmune diseases. Neutrophil- and monocyte-mediated inflammation is known to be involved in a number of human clinical manifestations, including acute leukocyte-mediated lung injury, such as adult respiratory distress syndrome (ARDS), multi-organ failure, reperfusion injury of myocardial and other tissues, such as liver, acute glomerulonephritis, reactive arthritis, various dermatoses, inflammatory disorders of central nervous system (CNS), such as acute purulent meningitis, thermal injury, inflammatory bowel disease, including ulcerative colitis and Crohn's disease, hemodialysis, leukapheresis, and cytokine-induced toxicity. Other disorders treatable in accordance with the present invention include traumatic shock, septic shock, frost-bite injury or shock and asthma. In addition, tumor metastasis can be inhibited or prevented by inhibiting the adhesion of circulating cancer cells, in particular in the case of hematogenous cancers. Examples include carcinoma of the colon and melanoma.

The CD34 polypeptides and antibodies capable of binding native CD34 of the patient to be treated are capable of blocking the adhesive interactions between leukocytes (including lymphocytes, neutrophils and monocytes) and the endothelium, and can thereby inhibit pathologic responses associated with leukocyte homing. For example, such agents can be used to inhibit the adhesion of neutrophils to the endothelium adjacent to the inflamed region. The CD34 polypeptides block the extravasation of neutrophils from blood to inflammatory sites via competitive binding to L-selectin expressed on the surface of neutrophils. The anti-CD34 antibodies inhibit neutrophil traffic to the site of inflammation by specific binding to native CD34 on endothelial cells.

The CD34 polypeptides and anti-CD34 antibodies of the present invention can be conveniently administered in conjunction with other pharmaceutically active agents, such as antiinflammatory agents, antioxidants, and further inhibitors of leukocyte adhesion to endothelial cells. Such further inhibitors may be soluble forms of other leukocyte cell adhesion molecules and their ligands, including integrins, integrin ligands, other selectins (e.g. E-selectin), other selectin ligands, e.g. GlyCAM-1, and carbohydrate L-selectin ligands, and antibodies to these adhesion molecules or their protein ligands. For example, the integrins CD11a/18 (LFA-1) and CD11b/18 (Mac-1) are known to be essential for neutrophil interactions with high endothelial venules (HEV), and for neutrophil localization to non-lymphoid sites of acute inflammation. Accordingly, the CD34 polypeptides and anti-CD34 antibodies herein can be administered in conjunction with compounds capable of antagonizing the interaction of CD11/18 with their endothelial ligands (ICAM-1, ICAM-2, ICAM-3). Such antagonists may, for example, be soluble derivatives of CD11, CD18, CD11/CD18 heterodimer, ICAM-1, ICAM-2, or ICAM-3, their fragments, and antibodies capable of selective binding CD11, CD18, CD11/CD18 heterodimer or the corresponding ICAM endothelial ligands. The various inhibitors of leukocyte adhesion may be covalently linked to yield molecules with multiple (usually dual) specificities.

The purified CD34 polypeptides and anti-CD34 antibodies of the present invention, including bispecific molecules comprising such moieties, may be administered as pharmaceutical compositions, usually formulated in dosage forms by methods known in the art; for example, see Remington's Pharmaceutical Sciences, Mack Publishing Company, Easton, Pennsylvania, 19th Edition 1985. For parenteral administration, the antagonists are typically formulated in the form of injectable solutions, suspensions or emulsions, in admixture with a suitable pharmaceutically acceptable vehicle and optionally other pharmaceutically acceptable additives. Typical vehicles include saline, dextrose solution, Ringer's solution, etc., but non-aqueous vehicles may also be used. For certain indications controlled release preparations may be preferred, which may, for example, be prepared by incorporating the active compounds of the present invention into particles of suitable polymeric material, or by entrapping them in microcapsules, liposomes, microemulsions, etc., such as, for example, disclosed in Remington's Pharmaceutical Sciences, supra. For a brief review of present methods for drug delivery, see, e.g. Langer, Science 249, 1527-1533 (1990).

The CD34 polypeptides or anti-CD34 antibodies of the present invention are administered to recipients in an amount sufficient to prevent or suppress the indicated physiological condition or symptom, e.g. inflammation. Accordingly, administration may take place prior to the onset or after the initiation of inflammation.

Administration is in conventional routes, including intravenous, intramuscular, subcutaneous, enteral and parenteral administration.

The effective dose may, of course, vary depending on such factors as the patient's age, weight, general medical condition, medical history, etc., and its determination is well within the skill of a physician. The effective dose generally is within the range of from about 1 pg/kg of body weight to about 10 mg/kg of body weight, more preferably 0.001 - 1 mg/kg of body weight.

In a particular embodiment, the anti-CD34 antibodies of the present invention can be used to target conventional anti-inflammatory drugs or other agents to specific sites of tissue injury. By using an anti-CD34 antibody to target a pharmaceutically active agent to lymphoid or extra-lymphoid, e.g., vascular endothelial cells, such agents can achieve higher local concentrations at the site of injury. This permits the alleviation of side-effects of conventional anti-inflammatory agents by lowering their overall dosage. Targeting can be achieved by covalently linking the anti-CD34 antibody to a pharmaceutically active moiety, or, for example, by making liposomes filled with a drug, e.g. an antiinflammatory agent, which incorporate the anti-CD34 antibody in the lipid membrane.

Further details of the present invention will be illustrated by the following non-limiting example.

### IV. EXAMPLES

### Example 1

### Identification of a second L-selectin ligand

### Materials and Methods

*Purification and amino acid sequence analysis of the Sgp 90 L-selectin ligand.* In order to demonstrate that isolated Sgp 90 has ligand activity for L-selectin, ³⁵S-sulphate labeled ligands for L selectin were prepared as previously described [Imai et al., J. Cell., Biol. 113, 1213-1221 (1991); Imai *et al.,* Glycobiology 2, 373-381 (1992); and Imai *et al.,* Nature 361, 555-557 (1993)], and eluted from an L selectin-IgG affinity column (substituted at 10 mg chimeric protein per ml of gel) with 5 mM EDTA in 0.5% Triton X-100 in tris-buffered saline. The eluate was subjected to reducing SDS-PAGE and the positions of Sgp50 and Sgp90 determined by autoradiography. The Sgp90 band was excised, rehydrated and electroeluted into 195 mM glycine, 25 mM tris, 0.01% SDS, 10% glycerol (5 mA, 12 hrs), and exchanged into 50 mM n-octylglucoside in PBS on a Centricon 30. The resulting solution (300 microliters) was split into 3 equal parts and subjected to reprecipitation with 25 microliters L selectin-IgG (LEC-IgG) in the absence or presence of 5 mM EDTA, or a CD4-IgG control matrix. Both supernatant and bound material were analyzed by 10% SDS-PAGE under reducing conditions, followed by autoradiography.

In order to purify Sgp90 for amino acid sequencing, the mesenteric lymph nodes from 1,965 mice were pooled and extracted with 2% Triton X-100 in PBS. The homogenized extract was clarified by centrifugation and was spiked with 40,000 cpm of ³⁵Sulphate-labeled PLN extract as previously described [Lasky *et al.,* Cell 69, 927-938 (1992)]. This mixture was boiled for 12 minutes, and the precipitated proteins removed by centrifugation. The supernatant was bound to a wheat germ agglutinin column and bound proteins were eluted in 0.2 M N-acetyl-glucosamine, 0.1% Triton. The eluted material was passed over a 40 mg L selectin-IgG affinity column and the bound fraction was released with EDTA. This affinity process was repeated, and the final EDTA released fraction was concentrated on a Centricon 30 and run on a single lane of an SDS 10% polyacrylamide gel. Proteins were electrophoretically transferred onto a ProBlott membrane and autoradiographed as previously described (Lasky *et al*., supra). The N-terminal amino acid sequence was determined using a model 470A Applied Biosystems sequencer equipped with an on-line PTH analyzer. Peaks were integrated with Justice Innovation software using Nelson Analytical 760 interfaces. Internal peptide sequence was obtained after tryptic digestion of the ProBlott membrane [Wong *et al.,* in Techniques in Protein Chemistry IV, R.H. Angeletti, ed., in press]. The 90 kD region of the matrix was wetted with 1 microliter of methanol. The blot was reduced with 100 microliters of 0.5 M Tris-HCl, pH 8.5, 10% acetonitrile, 5 mM EDTA and 7 mM dithiothreitol for 1 hr at 45 °C. The solution was cooled and 10 microliters of 200 mM iodoacetic acid in 0.5 M NaOH were added. The alkylation reaction continued for 20 minutes in the dark. The blot was then incubated with 200 microliters of 0.25% PVP-40 in 0.5 M acetic acid on a shaker for 20 minutes at room temperature. The residual PVP-40 was removed by rinsing the blot with water and then 20% acetonitrile. The 90 kD region was digested in 50 microliters of 0.1 M Tris-HCl, pH 8.0 containing 10% acetonitrile with 0.2 micrograms of Promega modified trypsin at 37 °C for 17 hrs. The supernatant was removed and the peptides were separated by capillary HPLC [Henzel et al., Anal. Biochem. 187, 228-233 (1990)] and the peptides were fractionated on a C18 0.32 x 150 mm capillary column (LC Packing, Inc.). The HPLC was performed with an Applied Biosystems 140A microgradient pump system and a model 783 UV detector equipped with a Z-shaped flow cell. The pump operated at 50 microliters/min. and was connected to a Valco tee used as a splitter to reduce flow to 6 microliters/min. Solvent A was 0.1% aqueous TFA and B was acetonitrile containing 0.8% TFA. Peptides were eluted using a linear gradient of 0-70% B in 50 minutes and detected at 195 nm. Several of the peptides were sequenced, but all gave very weak signals except for peptide 9 which gave a clearly interpretable sequence.

*Immunohistochemical staining of murine PLN with anti-CD34 polyclonal antiserum*. Recombinant mCD34 was produced as an IgG chimera as previously described [Watson et al., J. Cell., Biol. 110, 2221-2229 (1990)]. A cDNA of mCD34 [Bworn et al., H. Int. Immunol. 3, 175-184 (1991)] that terminated at lysine₂₈₆ was ligated to the hinge, CH2 and CH3 domains of human IgG⁵. This construct was transfected into human embryonic kidney (293) cells, and a permanent cell line producing mCD34-IgG was isolated. Serum free supernatants conditioned by this cell line were concentrated and passed over protein G sepharose to purify the mCD34-IgG chimera. The purified chimera was cleaved with immobilized papain (Pierce), and the extracellular domain of mCD34 was separated from the bulk (~80%) of contaminating human IgG1 Fc by protein A-Sepharose chromatography. Antiserum specific for mCD34 was produced by immunization of rabbits at multiple sites with ~100 micrograms of recombinant mCD34 in complete Freund's adjuvant. Rabbits were boosted with recombinant mCD34 in incomplete Freund's adjuvant. Anti-mCD34 titers were determined in an ELISA assay using purified mCD34-IgG chimera coated onto microtitre plates as previously described ⁵. The resultant polyclonal antisera were depleted of residual anti-human IgG antibodies by passage over a human IgG affinity column. 5-6 micron cryostat sections were cut from frozen murine PLN and transferred onto Vectabond-coated microscope slides. Sections were air dried for 30 minutes, fixed in cold acetone (10 minutes, 4°C) and again air dried (30 minutes). The sections were then rehydrated with 100 microliters of Dulbecco's phosphate buffered saline (D-PBS) for 10 minutes, and non-specific binding was blocked by incubation with D-PBS containing 0.1% BSA and 300 micrograms/ml goat IgG (blocking buffer) at 4°C for 20 minutes. Sections were then stained with a 1:100 dilution of rabbit anti-mCD34 antiserum in blocking buffer in the presence or absence of 100 micrograms/ml of mCD34-IgG chimera. After 1 hr at 4°C, the primary antibody was washed off by briefly dipping the slides twice into cold D-PBS. A 1:500 dilution of goat anti-rabbit IgG-HRPO conjugate (Caltag) was added for 30 minutes at 4°C, after which the sections were washed twice in D-PBS and distilled water prior to the addition of peroxidase chromogen (AEC, Biomeda Corp.). Color development proceeded for 20 minutes at room temperature after which time the slides were washed, counterstained with hematoxylin for 1 minute, air dried and visualized.

*Immunoprecipitation analysis of sulphate-labeled glycoproteins from murine PLN.* PLN were labeled with ³⁵S sulphate in organ culture as previously described (Imai et al., 1990, 1991, 1992, supra; Lasky *et al.,* 1992, supra). The Triton X-100 extract was precleared with protein G-Sepharose beads overnight at 4°C. The beads were removed, and the supernatant was divided into three aliquots. Immunoprecipitation was done with 10 microliters of rabbit anti-mCD34 antiserum or 10 microliters of rabbit anti-GlyCAM 1 antiserum (Lasky *et al.,* supra) in the presence of 50 microliters of packed protein G-Sepharose beads, or 50 microliters of L selectin-IgG conjugated Sepharose beads for 5 hrs at 4°C. The antibody-bound material was washed 4 times and electrophoresed on 4-20% SDS polyacrylamide gradient gels after boiling in SDS-beta mercaptoethanol Material bound to the L selectin-IgG coated Sepharose beads was eluted by incubation in PBS, 0.02% NP40, 0.05% triton X 100, 5 mM EDTA overnight at 4°C. 10 mM CaCl₂ was added to the eluted material, and separate aliquots were reprecipitated with either anti-GlyCAM 1 antiserum, anti-mCD34 antiserum or L selectin-IgG sepharose beads as described above.

Our previous studies revealed that Sgp90 is present in much smaller biochemical quantities than Sgp50 and is bound tightly to the endothelial cell surface (Imai *et al.* 1991, supra) in contrast to the Sgp50 ligand that is loosely associated with the endothelial cell and could be purified from conditioned medium [Lasky et al. 1992, supra; Brustein *et al.,* J. Exp. Med. 176, 1415-1419 (1992)]. Isolated Sgp50 was previously shown to interact with L selectin-IgG in a calcium-dependent manner. To prove that Sgp90 has ligand activity and is not merely co-precipitated with Sgp50, we electroeluted Sgp90 from an SDS gel and examined its ability to interact with L selectin-IgG. As shown in figure 1A, the eluted Sgp90 was quantitatively precipitated by L selectin-IgG in the presence of calcium, whereas a CD4-IgG chimera failed to react with the component. A procedure for the purification of Sgp90 from detergent lysates of mouse mesenteric lymph nodes was devised that incorporated a boiling step and affinity chromatography on wheat germ agglutinin and L selectin-IgG. The addition of ³⁵S-sulphate labeled PLN extract enabled monitoring of the purification¹⁰. The final EDTA-released fraction from the L selectin-IgG column was electrophoresed on an SDS-polyacrylamide gel, transferred to ProBlott membranes, and the Sgp90 ligand was localized by autoradiography. This region of the gel was isolated and subjected to amino acid sequence analysis.

Figure 1 B shows the results of the sequence analysis. A weak (~5 pM) 12 residue N-terminal sequence was determined that contained a number of gaps ("X"). Comparison of the N terminal sequence of the L selectin-purified Sgp90 ligand with the deduced N terminus of the murine sialomucin CD34 (mCD34) [Brown et *al.,* H. Int. Immunol. 3, 175-184 (1991)] revealed a striking relationship. The N terminus of the Sgp90 ligand matched the mCD34 N terminus at 7 out of 12 positions. Four of the positions that did not match were gaps in the Sgp90 sequence and threonines or serine in the mCD34 sequence. Many of the threonine and serine residues on CD34 are O-glycosylated [Simmons *et al*., J. Immunol. 148, 267-271 (1992); Brown et *al*., supra; Sutherland *et al.,* Leukemia 2, 193 (1988)], and the lack of interpretable signal at these positions in the amino acid sequence of the isolated Sgp90 ligand would be consistent with O-glycosylation of these sites. The final sequence difference was a threonine in the sequence of the Sgp90 L selectin ligand and a serine in the mCD34 sequence, a conservative substitution that could be explained by polymorphism. Additional evidence for the correspondence of the two proteins was obtained from the analysis of a tryptic peptide of the isolated ligand (Figure 1B). This sequence matched with mCD34 in all 6 positions, and it was preceded by a lysine in the mCD34 sequence, as would be expected for a tryptic fragment. This sequence analysis suggested that Sgp90 might be identical with mCD34.

In order to explore further the relationship of Sgp90 to mCD34, a polyclonal antiserum against mCD34 was produced. The recombinant antigen used for immunization of rabbits was a highly purified mCD34-IgG chimera that was subjected to papain cleavage to remove the majority (~80%) of the human IgG-1 constant domain. The purity of the injected material was determined by N-terminal sequencing. The resultant polyclonal anti-mCD34 antiserum was found by FACS analysis to specifically stain rat NRK cells that were stably transfected with a full length mCD34 cDNA, and a protein of approximately 90 kD is immunoprecipitated from these mCD34 transfectants (data not shown). Furthermore, the polyclonal antiserum specifically reacts with NIH3T3 cells known to express CD34 as well as with a small fraction of fetal liver and bone marrow progenitor/stem cells capable of reconstituting lethally irradiated mice (C. Jordan, S. Baumhueter and W. Matthews-unpublished observations). The latter finding is consistent with the localization of human CD34 on hematopoietic progenitor cells [Berenson *et al*., J. Clin. Inv. 81, 951(1988)].

The antiserum stained capillaries and the apical aspect of HEV within mouse PLN. This result is consistent with previous data that demonstrate the capillary and venular endothelial expression of human CD34 [Fina *et al.,* Blood 75, 2417-2426 (1990)], and it extends these data by demonstrating that this antigen is also expressed in the microvasculature of PLN, a lymphoid site that is known to be dependent upon adhesive interactions mediated by L selectin [Stoolman, L., Cell 56, 907-910 (1989); Springer, T.A., Nature 346, 425-434 (1990); Butcher, E.C., Cell 67, 1033 (1991); Lasky, L.A., Science 258, 964-969 (1992)]. Importantly, the L selectin-IgG chimera, when used as a histochemical reagent, also stains PLN HEV [Watson *et al.,* J. Cell. Biol. 110, 2221-2229 (1990)].

To directly test whether the antiserum against mCD34 reacted with Sgp90, an immunoprecipitation analysis was performed (Figure 3). Sgp50 and Sgp90 were precipitated from a sulphate labeled lymph node lysate with L selectin-IgG and eluted with EDTA (Imai *et al*. 1991, 1992, 1992, supra) A peptide antibody against Sgp50 (i.e. GlyCAM 1) (Lasky *et al.,* supra) selectively precipitated Sgp50 but not Sgp90. In contrast, the polyclonal anti-mCD34 serum precipitated the Sgp90 component almost quantitatively while not affecting Sgp50. These data, together with the immunohistochemical analysis of Figure 2, confirm that CD34 is a sulphated endothelial-associated glycoprotein in PLN and that this 90kD molecule is identical to the previously described L selectin ligand known as Sgp90. It remains to be determined what the relationship of mCD34, and hence Sgp90, is to the ~90kD component recognized (among a complex set of glycoproteins) by the MECA-79 monoclonal antibody, an adhesion-blocking antibody that stains PLN HEV [Berg *et al.,* J. Cell. Biol. 114, 343-349 (1991)].

The foregoing results demonstrate that a second, mucin-like glycoprotein (i.e. CD34) can function as an endothelial-associated ligand for L selectin. As Figure 4 illustrates, the hypothetical structures of GlyCAM 1 (Sgp50) and CD34 (Sgp90) show a number of similarities and differences. The most compelling similarity is the prominence of highly O-glycosylated, mucin-like domains in both proteins. We previously proposed that such mucin-like domains, which are predicted to be highly rigid and extended structures [Jentoft, N., Trends Biochem. Sci. 15, 291-294 (1990)], can function as scaffolds for the polyvalent presentation of the appropriate, sulphated and sialylated carbohydrate ligands to the L selectin lectin domain. Thus, CD34, a known endothelial sialomucin, is uniquely qualified, both in terms of structure and endothelial localization, to serve as a scaffold for carbohydrate presentation to L selectin on opposed leukocytes. However, it should also be noted that the structure of CD34 differs from that of GlyCAM 1 in several interesting respects. For example, CD34 is directly attached to the cell surface by a conventional transmembrane motif, consistent with the requirement for detergent extraction during ligand purification (Figure 1), while GlyCAM 1 does not appear to contain such an anchoring motif and is peripherally associated with the membrane. Furthermore, Sgp90 partitions quantitatively into liposomes while Sgp50 remains in the aqueous phase (S. Hemmerich and S. Rosen, unpublished). In addition, CD34 appears to contain a number of other motifs, including a cysteine-rich region as well as an extended cytoplasmic domain that may have a role in signaling during leukocyte adhesion.

Another important difference between GlyCAM 1 and CD34 is their tissue distribution. (Figure 5). GlyCAM 1 is expressed predominantly in the high endothelial venules of PLN, consistent with its proposed function as an endothelial ligand for L selectin-mediated trafficking to this lymphoid organ (Stoolman, L. 1989, supra), while vascular CD34 has a much broader tissue distribution. For example, human CD34 appears to be expressed in a wide diversity of capillary and post capillary venule endothelial cells (Fina *et al.,* supra) and northern blot [Bworn *et al.,* H. Int. Immunol. 3, 175-184 (1991)] and PCR analysis (S. Baumhueter-unpublished observations) suggests a similar pattern for mCD34. It is believed that extra-lymphoid vascular CD34 could function as an L selectin adhesive ligand that is utilized during acute and chronic inflammatory responses. Previous work has demonstrated an important role for neutrophil L selectin during the initial, low affinity rolling response that is a prerequisite for the higher affinity integrin-mediated adhesion and extravasation events during inflammation. The broad distribution of extra-lymphoid vascular CD34 on endothelial cells, together with the ability of the PLN HEV form to serve as an L selectin ligand, are suggestive of a function for the extra-lymphoid CD34 in neutrophil rolling mediated by L selectin [Ley, K. *et al.,* Blood 77, 2553-2555 (1991); von Adrian *et al.,* Proc. Natl. Acad. Sci. USA 88, 7538-7542 (1991)]. Because of the apparently constitutive nature of the expression of vascular CD34 (Fina *et al*., supra), various regulatory pathways, including changes in CD34 conformation, oligomerization, glycosylation or translocation to the endothelial cell surface, may determine the adhesive activity of CD34. Finally, in light of the present results, the localization of CD34 on hematopoietic stem cells, together with its lectin-dependent cell binding activity demonstrated here, suggests that stem cell CD34, possibly containing a distinctive pattern of glycosylation, may perform adhesive functions during early hematopoiesis, perhaps by interaction with a bone marrow stromal lectin.

### Example 2

### Expression of CD34 in various tissues

In Example 1 we have demonstrated the ability of PLN-derived CD34 to bind L-selectin, and provided data indicating that CD34 has a much broader range of tissue distribution than the other L-selectin ligand, GlyCAM-1.

In this example, we provide evidence that CD34 is expressed at vascular sites in all murine organs and tissues examined, a result which further accentuates the possibility that vascular CD34 is involved in L-selectin mediated neutrophil rolling. In addition, it will be shown that the vascular expression of CD34 is maintained at inflammatory sites, consistent with a potential role in directing leukocyte traffic. Finally, it will be demonstrated that, if vascular CD34 does not display the appropriate carbohydrate modifications, it does not appear to serve as an L-selectin ligand and is insufficient to support lymphocyte tafficking to PLN.

### Materials and Methods

Antibody *Production and Characterization*. Polyclonal antibodies against murine CD34 were prepared essentially as described in Example 1 and in Baumheuter, S. *et al.,* Science 262, 436-438 (1993). Briefly, a recombinant mCD34/IgG chimera was purified from transfected cell supernatants using protein G affinity chromatography. The material was then cleaved with immobilized papain (Pierce) as specified by the manufacturers instructions (3hrs at 37°C) and passed over a second protein G column to remove the human IgG Fc portion. 100 µg of the flow through containing the extracellular domain of CD34 was used to inoculate rabbits together with complete Freund's adjuvant. Antibody titres were determined in an ELlSA assay using recombinant CD34/IgG as immobilized antigen. Rabbit serum was first depleted of antihuman IgG antibodies by passage over a human IgG column and anti-CD34 antibodies were purified from the flow through on a CD34/IgG affinity column. Bound antibody was eluted with 0.1 M acetic acid/0.15M NaCl (pH3.0), immediately neutralized with 1 M Tris (pH 8.8), dialyzed against three changes of PBS and stored at -70° C in 10% Glycerol/PBS.

*Immunohistochemistry*. Immunohistochemistry was performed as described in Example 1 or in Baumheuter *et al.* (1993), supra, either on fresh frozen or periodate-lysine-paraformaldehyde (PLP) fixed tissue sections [15]. Briefly, 5-8 µm paraffin sections were deparaffinized in xylene for 10 minutes, rinsed in water, and endogenous peroxidase was quenched with 1% H₂O₂ for 30 minutes. For staining with the CD34 antibody sections were immersed in 0.1 M citrate buffer, pH6.0 and microwaved twice on high for 3 minutes followed by 20 minutes at room temperature. For staining with the anti GlyCAM 1 antibody the citrate antigen retrieval step was replaced by a 3 minute pepsin digestion at 37°C. Nonspecific binding was blocked by a 20-30 minute preincubation with 10% normal goat serum followed by a 30 minute room temperature incubation with the appropriate dilution of antibody. The sections were rinsed in PBS, incubated with biotinylated goat anti rabbit IgG (Vector Laboratories) for 30 minutes, rinsed in PBS, and incubated with incubated with the Vector Elite ABC reagents (streptavidin horseradish peroxidase conjugate) as specified by the manufacturer's instructions. Substrate (DAB, Dako) was added for 5 minutes. The sections were counterstained with Mayers' hematoxylin, dehydrated, and mounted in synthetic mounting medium. Modifications that were made for frozen tissue sections included the addition of an acetone fixation step and omission of the deparaffinization and antigen retrieval (citrate/pepsin) steps. Evaluation of staining was done using a scale from 0 to 3 where 3 represents strongest reactivity.

Staining of sections with L-selectin/IgG was done using a procedure modified from previous methods [Watson *et al.* (1990), supra; and Mebius et *al.,* J. Immunol. 151(12), 6769-6776 (1993)]. L-selectin/IgG was conjugated to gold particles as previously described [24]. 5-8 µm cryostat sections of PLP fixed, frozen tissue were placed onto Vectabond-coated slides, dried, fixed with cold acetone and dried. 100 of 200 µg/ml CD4 IgG containing 1% BSA in PBS was added to the slide and incubated for 15 minutes at 4°C on a rotating platform set at 70 rpm. Sections were then incubated with 100 µl of 50 µg/ml L-selectin/IgG chimera in the presence or absence of 10mM EGTA or with control CD4/IgG alone for 45 minutes at 4°C and 70 rpm. The sections were gently decanted, fixed with cold 2.5% gluteraldehyde in PBS for 30 minutes and washed in three changes of distilled water. L-selectin/IgG gold staining was developed using the IntenSE silver enhancement kit from Amersham. Sections were rinsed, counterstained with Mayers' hematoxylin and mounted in Crystal/Mount (Biomedia). Induction of Inflammatory Responses Mice were primed by spotting 25 µl of a solution containing 1 mg/ml oxazolone (Sigma) in 80% acetone/20% olive oil (v/v) on both hindlegs. Draining lymph nodes were harvested after 5 days and processed for immunohistochemistry as described above. An immediate type hypersensitivity response was induced by spotting 10 µl of the same oxazolone solution onto the right ears of primed mice on day 5. The left ears were treated with solvent only and served as control. Ears were harvested 3 hours after induction of the DTH response and processed for immunohistochemistry. Non obese diabetic (NOD) mice were obtained from Taconic, Germantown, New York and pancreases of male and female animals were harvested at 20 weeks after birth. The incidence of development of diabetes is approximately 80% in female and 30% in male mice at the age of 24 weeks. Deafferentiation of PLN. Deafferentiation was done as described in Mebius *et al.,* supra. Briefly, popliteal lymph nodes were exteriorized, and afferent lymphatics were severed, leaving the efferent lymphatics and blood vessels intact. Mice were euthanized 1 week after deafferentiation. The completeness of deafferentiation was determined by injection of 50 µl of a 10% India ink solution into the ipsilateral footpad. Lymph nodes with intact afferent lymphatics, as determined by uptake of India ink, were discarded. The contralateral popliteal lymph node served as the unoperated control.

### Results

### Production of a specific polyclonal antibody directed against murine CD34

High titre antibody production against murine CD34 was achieved using a recombinant form of this protein purified from the supernatant of stably transfected mammalian cells [Brown *et al*., Int. Immunol. 3, 175-184 (1991)]. The hinge, CH2 and CH3 domains of human IgG-1 were attached to the extracellular domain of murine CD34 (mCD34/IgG) (Fig. 6A) [Watson *et al.* (1990), supra] and this construct was transfected into human embryonic kidney (293) cells. Purification of the mCD34/IgG on a protein G sepharose column enabled the isolation of milligram quantities of the antigen with a molecular weight of ~90 kD (Fig. 6B). In order to enhance the antibody response to the murine CD34, the bulk of the human IgG-1 Fc was removed by digestion with immobilized papain followed by protein G sepharose chromatography. The resultant flow through from this column contained approximately 80% pure extracellular domain of CD34 which was used for immunization of rabbits.

The anti-CD34 antibody was immunoaffinity purified from rabbit serum using immobilized recombinant mCD34/IgG and tested for recognition of native CD34 in a number of systems. Figure 7 illustrates that the antibody specifically recognizes cell surface CD34 on rat NRK cells that were transfected with a full-length CD34 expression construct (Fig. 2A) and also recognizes NlH3T3 cells which were shown to express high levels of CD34 mRNA (Fig. 7B).

Immunoprecipitation analysis of the CD34 transfectants as well as of NIH 3T3 cells showed a band at 100 kD that was not seen using preimmune serum (data not shown). In addition, previous immunohistochemical analysis demonstrated that this polyclonal antiserum specifically recognized capillaries and HEV in murine PLN [Example 1 and Baumhueter *et al.* (1993), supra]. Finally, this antiserum has been used for the isolation of hematopoietic stem cells from murine bone marrow and fetal liver (C. Jordan, S. Baumhueter, L. Lasky and W. Matthews-unpublished data). Taken together, these data suggest that the affinity purified anti-CD34 antiserum is a highly specific reagent for the detection of murine CD34 in biochemical and histological experiments.

### Distribution of CD34 in normal mouse tissues

The distribution of CD34 in murine tissues was then investigated by immunohistochemistry. The results of this survey are summarized in Table 1. In all organs examined, prominent staining of capillary and post capillary venues was observed and Figure 8 illustrates the staining of vascular endothelium in brain (Fig. 8A), kidney (Fig. 8B) and thymus (Fig. 8C) and the staining of megakaryoblasts and a small percentage of blast-like cells, most likely hematopoietic progenitors, in bone marrow (Fig.8D). Staining was always lumenally oriented with the exception of megakaryoblasts where it was apparently cytoplasmic. In some tissues, e.g. lymph nodes and thymus, capsules were reactive with the CD34 antibody which most likely recognizes endothelial cells in these structures (see also comments, Table 1). Taken together our data demonstrate the global vascular expression of murine CD34.

### CD4 expression on vessels at inflammatory sites

We examined the expression of CD34 in the murine system in PLN draining an inflammatory site, in the skin of mice undergoing a DTH response and in the pancreases of NOD mice undergoing an inflammatory response.

In all three models, we found that CD34 expression was maintained on vessels at the site of inflammation. CD34 expression on HEVs and capillaries of PLN draining a site of antigen was comparable to noninflamed nodes (Fig.9). No major up- or downregulation was apparent, however moderate modulation might not be detected using immunohistochemistry. When comparing skin from normal mice and mice undergoing an immediate type hypersensitivity response, vascular staining of CD34 was prominent in both cases (Fig. 9). Examination of pancreases of 20 week old female NOD mice undergoing a massive leukocyte infiltration showed no change in vascular CD34, and, interestingly, some newly developed capillaries and HEV like structures in the leukocyte infiltrate were also stained (Fig. 4). Thus, at least in these models of inflammation CD34 expression did not seem to be downregulated.

The finding that CD34 was expressed on HEV-like vessels in the inflammatory infiltrate in the female NOD pancreas prompted us to investigate the possible binding of L-selectin to these vessels. Previous data have demonstrated that HEV-like vessels, which are normally found in lymph nodes, are induced during the later stages of inflammation in NOD mice [Hanninen *et al.,* J. Clin. Invest. 92, 2509-2515 (1993); Yang *et al.,* Proc. Natl. Acad. Sci. USA 90, 10494-10498 (1992)]. These structures have been shown to also react with MECA79 and are positive for MadCAM 1. It therefore seemed of interest to demonstrate L-selectin binding to the HEVs in the NOD pancreas and to investigate whether there might be overlapping expression of GlyCAM 1 and CD34 that would suggest that either or both of these proteins could act as L-selectin ligands. Staining of NOD mouse pancreases with the L-selectin/IgG chimera revealed that at least a subset of these HEV-like vessels appear to specifically bind the chimera (Fig. 10A, B) in a calcium dependent manner. Serial sections show that the same vessels are positive for GlyCAM and CD34 (Fig. 10C, D). No L-selectin staining could be detected in the control male NOD pancreas or the pancreas of normal mice (not shown). Since, under normal conditions, GlyCAM 1 appears to be present only on PLN and MLN HEV, these data demonstrate that GlyCAM 1 and L-selectin/IgG binding can be induced by chronic inflammation in non-lymphoid sites. This suggests that the induced HEV-like vessels in the inflamed NOD pancreas are phenotypically similar to PLN HEV. Furthermore our results demonstrate that the mere presence of vascular CD34 is insufficient for L-selectin/IgG binding, a finding that is consistent with a role for specific carbohydrate modifications, such as sulfation, in high affinity recognition of CD34 by L-selectin.

### Expression of L-selectin ligands in deafferentiated PLN

Previous data have demonstrated that interruption of afferent lymphatic flow results in a profound decrease in the trafficking of lymphocytes to PLN in vivo with the result that such treated PLN become significantly smaller in size [Mebius *et al.,* Mebius *et al*., J. Cell. Biol. 115, 85-95 (1991)]. Microscopic analysis of these deafferentiated PLN shows that the normally high walled venules (HEV) that appear to mediate lymphocyte attachment via L-selectin become flat walled. Furthermore, lymphocyte adhesion to these flat walled endothelial cells in vitro appears to be greatly diminished and staining of the deafferentiated PLN with the monoclonal antibody MECA 79, which has been shown to recognize a sulfated carbohydrate epitope on a number of PLN HEV glycoproteins [Imai *et al*., *J.* Cell. Biol. 113, 1213-1221 (1991)] and which blocked the L-selectin dependent binding of lymphocytes to PLN HEV, showed a profound decrease. Finally, recent data have demonstrated that an L-selectin/IgG chimeric protein is unable to bind to the HEV in deafferentiated PLN [Mebius *et al.,* (1993), supra]. This study also demonstrated that both the mRNA for GlyCAM 1 as well as the sulfated glycoprotein itself were undetectable in the treated PLN. It was, therefore, of great interest to determine the expression of mCD34 in these treated organs.

As can be seen in Figure 11, deafferentiation downregulates the expression of vascular GlyCAM 1. Surprisingly the expression of CD34 is unchanged in the deafferentiated PLN, despite the fact that these nodes are negative for MECA 79 and L-selectin/IgG staining. The continued expression of CD34 in the absence of L-selectin binding again underlines the importance of appropriate posttranslational carbohydrate modifications for high affinity L-selectin binding. The protein backbone alone without the relevant sulfated carbohydrate ligand is insufficient to mediate lymphocyte trafficking to PLN. Our results also suggest that there are different levels of regulation of the two L-selectin ligands following deafferentiation, i.e. downregulation of GlyCAM 1 expression versus regulation of carbohydrate modification of CD34 with maintenance of protein expression.

### Discussion

The global vascular distribution of mCD34 reported here is consistent with the possibility that it functions as an endothelial ligand for L-selectin dependent adhesion of neutrophils to non-lymphoid sites. The constitutive expression of mCD34 at all vascular sites may be viewed as counterintuitive in light of the fact that neutrophils do not roll along vessels in the absence of an inflammatory insult. Recent information gleaned from mice that have been made null for P-selectin may help to explain this apparent conundrum. These mice were found to be deficient in both neutrophil rolling along mesenteric venues and neutrophil influx into the peritoneum in response to the inflammatory stimulant, thioglycollate [Mayadas *et al.,* Cell 74, 541-554 (1993)]. Thus, the most obvious conclusion from these mutant mice was that P-selectin was a critical component of neutrophil rolling and influx. Because previous investigations have shown that L-selectin is also apparently critical for neutrophil rolling and extravasation, it seems likely that both of these adhesion molecules are necessary for neutrophil influx, but neither protein alone is sufficient to mediate this process. The acute nature of neutrophil inflammation would require that adhesion molecules involved in neutrophil rolling be constituitively expressed, and the constitutive lumenal expression of vascular CD34 and granular expression of P-selectin fulfill this criterion. The physiological reason for requiring both L- and P-selectin during neutrophil rolling can only be speculated upon. For example, it is possible that one of the selectins may serve to initiate the interaction while the other may strengthen the binding event.

Another interesting aspect to the present data derives from the indirect demonstration that CD34 must be glycosylated differently in PLN and MLN versus nonPLN or MLN vessels. Previous data have demonstrated that the MECA 79 monoclonal antibody recognizes a carbohydrate epitope on a number of endothelial glycoproteins [Streeter *et al*., J. Cell. Biol. 107, 1853-1862 (1988); Berg *et al.,* J. Cell. Biol. 114, 343-349 (1991)], one of which is CD34 [Imai *et al.,* J. Cell. Biol. 113, 1213-1221 (1991)]. This epitope is predominately expressed in PLN and MLN HEV, demonstrating that the carbohydrate recognized by MECA 79 is expressed in only a small subset of vascular endothelial cells. In addition, the MECA 79 antibody blocked the L-selectin mediated adhesion of lymphocytes to PLN HEV in a frozen section assay, suggesting that this carbohydrate might be a component of the carbohydrate ligand recognized by the lectin domain of L-selectin [Streeter *et al.,* supra]. The finding that CD34 is expressed on vascular sites that do not show MECA 79 antigen expression therefore suggests that non-PLN CD34 has a different pattern of carbohydrate modification than does PLN CD34. This result does not, however, necessarily indicate that the non-PLN CD34 is unable to bind to L-selectin. For example, it is possible that the carbohydrate modification of PLN CD34 detected by MECA 79 serves to enhance the binding efficiency of lymphocytes as they traverse through these organs, while a lower avidity interaction between neutrophils and non-PLN CD34 lacking the MECA 79-specific carbohydrate might result in the rolling response of this leukocyte type. This latter hypothesis is strengthened by the finding reported here that CD34 expression is maintained on deafferentiated PLN vessels, in spite of the fact that lymphocyte trafficking to these organs is significantly decreased. The previously described lack of MECA 79 expression on the venules of deafferentiated PLN again suggests that vascular CD34 can be differentially glycosylated under various physiological states. In addition, the demonstration that L-selectin/IgG only appears to bind to vessels in inflamed NOD pancreases that are HEV-like, i.e. that express GlyCAM 1 and presumably MECA 79, is also consistent with the hypothesis that differential glycosylation of vascular CD34 may modulate the strength of L-selectin binding.

In conclusion, the data reported here are consistent with a role for non-PLN vascular CD34 in L-selectin dependent trafficking to a diversity of organs and tissues in the mouse. While it may seem possible to test this hypothesis by the production of antibodies directed against mCD34 that block L-selectin dependent adhesion, the carbohydrate nature of the L-selectin ligand presented by mCD34 might make such an undertaking difficult. This is due to the fact that anti-carbohydrate antibodies often lack specificity and are usually of relatively low avidity. An alternative approach might, therefore, be to produce a strain of mice with a null mutation in the CD34 gene. Such investigations will undoubtedly further our understanding of the various adhesion systems that are utilized during acute and chronic inflammation.

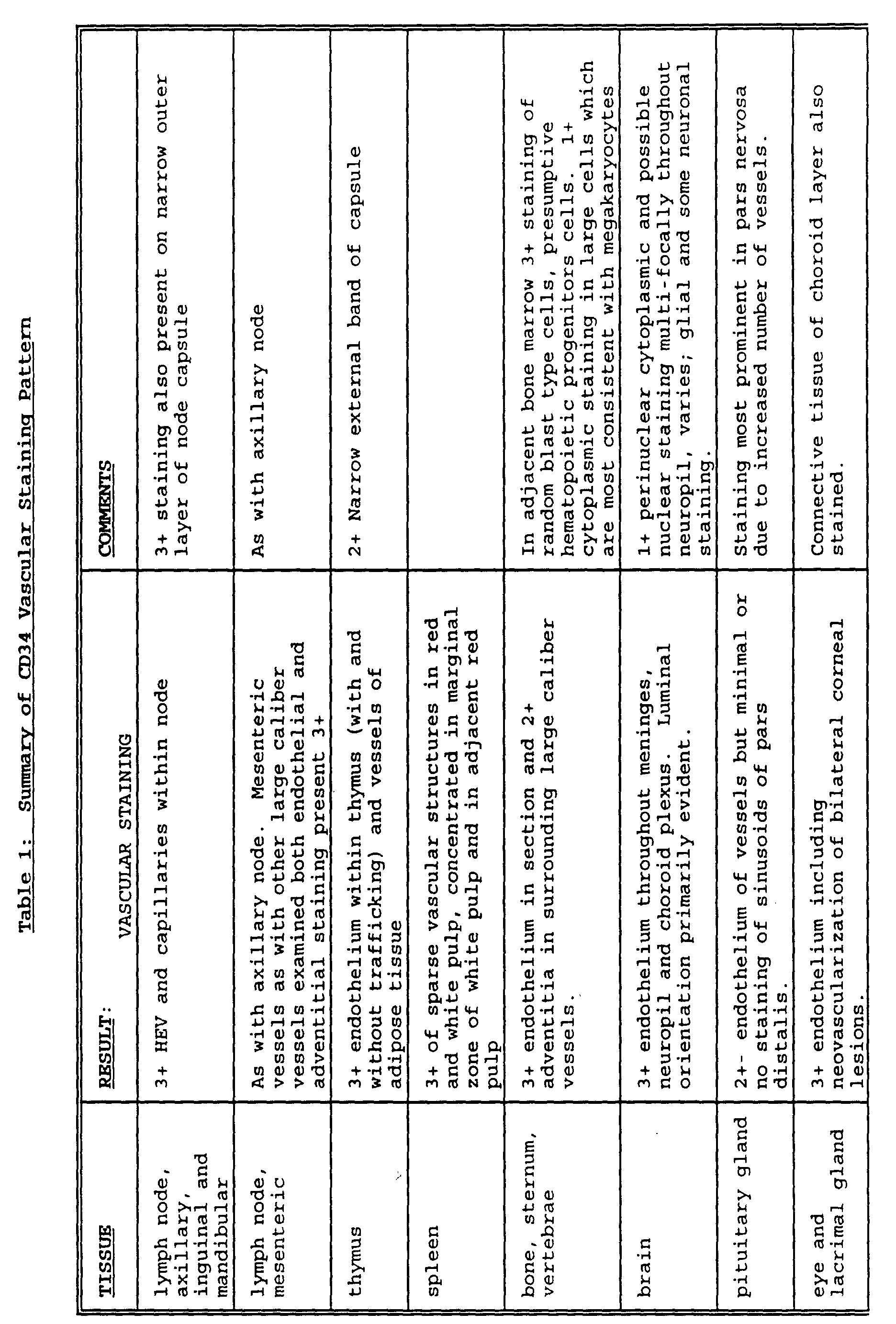

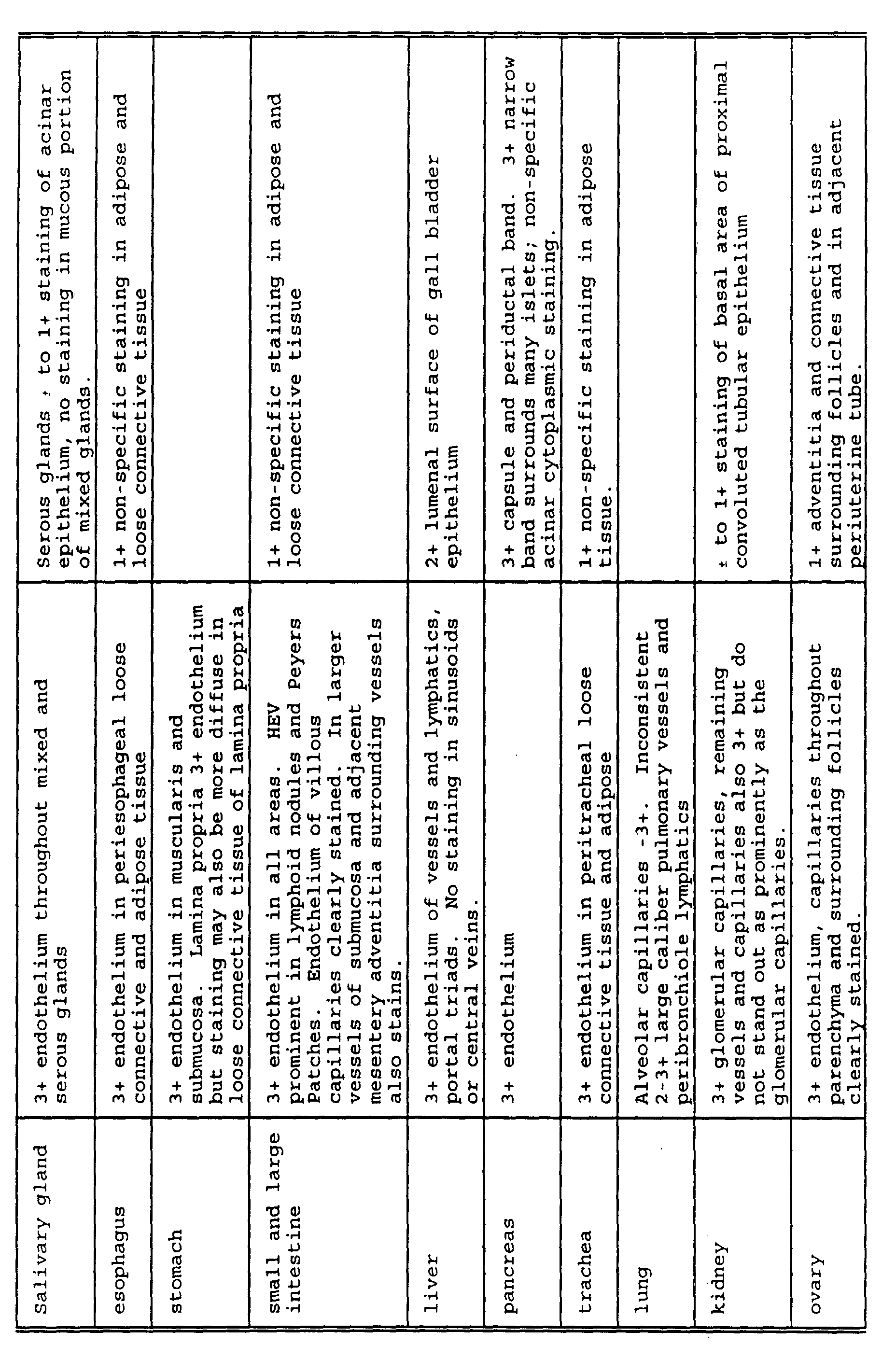

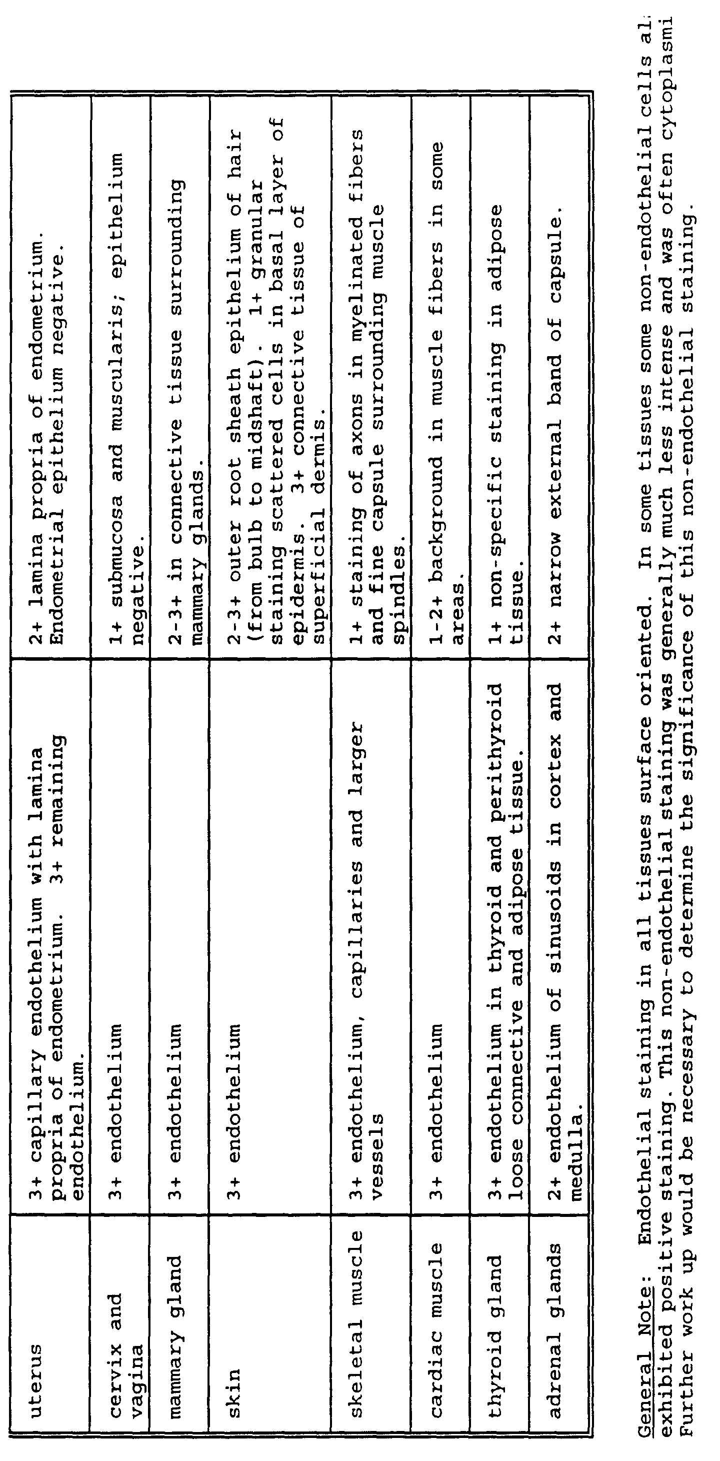

Although the foregoing refers to particular preferred embodiments, it will be understood that the present invention is no so limited. It will occur to those ordinarily skilled in the art that various modification may be made to the disclosed embodiments without diverting from the overall concept of the invention. All such modifications are intended to be within the scope of the present invention

## Claims

1. An isolated, purified CD34 polypeptide or an antibody capable of binding a native CD34 for use in a method of medical treatment.

2. The use of an isolated, purified CD34 polypeptide or an antibody capable of binding a native CD34 in the preparation of a medicament for inhibiting a pathological condition associated with intercellular adhesion mediated by L-selectin.

3. The use of claim 2 wherein the pathological condition is associated with the adhesion of leukocytes to endothelial cells.

4. The use of claim 3 wherein the leukocytes are lymphocytes and the endothelial cells are on peripheral or mesenteric lymph nodes.

5. The use of claim 2 wherein the pathological condition is an autoimmune disease.

6. The use of claim 5 wherein the pathological condition is rheumatoid arthritis, multiple sclerosis, psoriasis, or chronic dermatitis.

7. The use of claim 3 wherein the leukocytes are neutrophils or monocytes, and the endothelial cells are those of venular endothelium.

8. The use of claim 7 wherein the pathological condition treated is acute or chronic inflammation.

9. The use of claim 7 wherein the pathological condition is adult respiratory distress syndrome (ARDS), multi-organ failure, reperfusion injury, acute glomerulonephritis, reactive arthritis, dermatosis, acute purulent meningitis, thermal injury, ulcerative colitis, Crohn's disease, hemodialysis, leukapheresis, hemorrhagic shock, or cytokine-induced toxicity.

10. The use of claim 3 further comprising the administration of a therapeutically effective amount of a compound selected from the group consisting of:
(a) a selectin;
(b) a selectin ligand other than a CD34 polypeptide;
(c) an antibody capable of binding a selectin or a selectin ligand other than a CD34 polypeptide;
(d) an integrin;
(e) an integrin ligand;
(f) an antibody capable of binding an integrin or an integrin ligand; and
(g) a non-protein antagonist of L-selectin-CD34 interaction.

11. The use of claim 10 wherein said compound is a P-selectin, a P-selectin ligand or an antibody capable of binding P-selectin.

12. The use of any one of claims 2 to 11 further comprising the administration of a steroidal or non-steroidal antiinflammatory agent.

13. The use of any one of claims 2 to 12 wherein said patient is a mammal, preferably a human.

14. The use of any one of claims 2 to 13 wherein said CD34 polypeptide has a carbohydrate structure recognized by the monoclonal antibody MECA 79.

15. The use of an antibody capable of binding a native CD34, wherein the antibody is chemically or physically associated with a pharmaceutically active compound, in the preparation of a medicament for targeting the pharmaceutically active compound to endothelial cells.

16. The use of claim 15 wherein said pharmaceutically active compound is an antiinflammatory agent or an antioxidant.

17. The use of claim 15 or claim 16 wherein said pharmaceutically active compound is directly fused to a constant domain sequence of said antibody.

18. A bispecific molecule comprising a CD34 sequence or an antibody sequence capable of binding a native CD34 and a further pharmaceutically active moiety.

19. The bispecific molecule of claim 18 comprising an antibody sequence capable of binding a native CD34 and a pharmaceutically active moiety of an antiinflammatory agent or antioxidant.

20. The bispecific molecule of claim 18 comprising a first antibody sequence capable of binding a native CD34 and a second antibody sequence capable of binding a different molecule associated with the leukocyte adhesion.

21. The bispecific molecule of claim 20 wherein said second antibody sequence is capable of binding a native selectin ligand other than CD34 or is capable of binding a native integrin ligand.

22. The bispecific molecule of claim 21 wherein said integrin ligand is a member of the ICAM family.

23. A pharmaceutical composition comprising an anti-CD34 antibody.

24. The pharmaceutical composition of claim 23 further comprising an additional pharmaceutically active compound.

25. Use of a carbohydrate antagonist of L-selectin-CD34 interaction in the preparation of a medicament for presenting said carbohydrate antagonist to endothelial cells expressing CD34, wherein said antagonist is attached to the polypeptide backbone of a CD34 polypeptide.

## Patentansprüche

1. Isoliertes, gereinigtes CD34-Polypeptid oder Antikörper, der ein natives CD34 binden kann, zur Verwendung in einem medizinischen Behandlungsverfahren.

2. Verwendung eines isolierten, gereinigten CD34-Polypeptids oder eines Antikörpers, der ein natives CD34 binden kann, zur Herstellung eines Medikaments zur Hemmung eines pathologischen Zustands, der von durch L-Selectin vermittelter interzellulärer Adhäsion begleitet ist.

3. Verwendung nach Anspruch 2, worin der pathologische Zustand von Adhäsion von Leukozyten an Endothelzellen begleitet ist.

4. Verwendung nach Anspruch 3, worin die Leukozyten Lymphozyten sind und die Endothelzellen auf peripheren oder mesenterischen Lymphknoten vorliegen.

5. Verwendung nach Anspruch 2, worin der pathologische Zustand eine Auto-immunerkrankung ist.

6. Verwendung nach Anspruch 5, worin der pathologische Zustand primärchronische Polyarthritis, multiple Sklerose, Psoriasis oder chronische Dermatitis ist.

7. Verwendung nach Anspruch 3, worin die Lymphozyten Neutrophile oder Monozyten ist und die Endothelzellen jene von Venülen-Endothel sind.

8. Verwendung nach Anspruch 7, worin der behandelte pathologische Zustand eine akute oder chronische Entzündung ist.

9. Verwendung nach Anspruch 7, worin der pathologische Zustand Respiratory-Distress-Syndrom bei Erwachsenen (ARDS), Mehrfach-Organversagen, Reperfusionsschädigung, akute Glomerulonephritis, reaktive Arthritis, Dermatose, akute eitrige Meningitis, Verbennungen, ulzeröse Kolitis, Morbus Crohn, Hämodialyse, Leukapherese, hämorrhagischer Schock oder zytokininduzierte Toxizität ist.

10. Verwendung nach Anspruch 3, weiter umfassend die Verabreichung einer therapeutisch wirksamen Menge einer Verbindung, die aus folgender Gruppe ausgewählt ist:
(a) einem Selectin;
(b) einem anderen Selectin-Liganden als einem CD34-Polypeptid;
(c) einem Antikörper, der ein Selectin oder einen anderen Selectinliganden als ein CD34-Polypeptid binden kann;
(d) einem Integrin;
(e) einem Integrin-Liganden;
(f) einem Antikörper, der ein Integrin oder einen Integrin-Liganden binden kann; sowie
(g) einem Nicht-Protein-Antagonisten der L-Selectin-CD34-Wechselwirkung.

11. Verwendung nach Anspruch 10, worin die Verbindung ein P-Selectin, ein P-Selectin-Ligand oder ein zur Bindung von P-Selectin fähiger Antikörper ist.

12. Verwendung nach einem der Ansprüche 2 bis 11, die weiters die Verabreichung eines Steroid- oder Nicht-Steroid-Entzündungshemmers umfasst.

13. Verwendung nach einem der Ansprüche 2 bis 12, worin der Patient ein Säugetier, vorzugsweise ein Mensch, ist.

14. Verwendung nach einem der Ansprüche 2 bis 13, worin das CD34-Polypeptid eine Kohlenhydratstruktur aufweist, die vom monoklonalen Antikörper MECA 79 erkannt wird.

15. Verwendung eines zur Bindung eines nativen CD34 fähigen Antikörpers, worin der Antikörper chemisch oder physikalisch mit einer pharmazeutisch aktiven Verbindung verbunden ist, zur Herstellung eines Medikaments, um die pharmazeutisch aktive Verbindung auf Endothelzellen auszurichten.

16. Verwendung nach Anspruch 15, worin die pharmazeutisch aktive Verbindung ein Entzündungshemmer oder ein Oxidationshemmer ist.

17. Verwendung nach Anspruch 15 oder 16, worin die pharmazeutisch aktive Verbindung direkt mit einer Konstantdomänensequenz des Antikörpers fusioniert ist.

18. Bispezifisches Molekül, das eine CD34-Sequenz oder eine Antikörpersequenz, die ein natives CD34 binden kann, und eine weitere pharmazeutisch aktive Gruppe umfasst.

19. Bispezifisches Molekül nach Anspruch 18, das eine Antikörpersequenz, die ein natives CD34 binden kann, und eine pharmazeutisch aktive Gruppe eines Entzündungshemmers oder Oxidationshemmers umfasst.

20. Bispezifisches Molekül nach Anspruch 18, das eine zur Bindung eines nativen CD34 fähige Antikörpersequenz sowie eine zweite Antikörpersequenz umfasst, die ein anderes Molekül binden kann, das mit der Leukozyten-Adhäsion in Zusammenhang steht.

21. Bispezifisches Molekül nach Anspruch 20, worin die zweite Antikörpersequenz einen anderen nativen Selectin-Liganden als CD34 oder einen nativen Integrin-Liganden binden kann.

22. Bispezifisches Molekül nach Anspruch 21, worin der Integrin-Ligand ein Mitglied der ICAM-Familie ist.

23. Pharmazeutische Zusammensetzung, die einen Anti-CD34-Antikörper umfasst.

24. Pharmazeutische Zusammensetzung nach Anspruch 23, weiters umfassend eine zusätzliche pharmazeutisch aktive Verbindung.

25. Verwendung eines Kohlenhydrat-Antagonisten der L-Selektin-CD34-Wechselwirkung zur Herstellung eines Medikaments, um den Kohlenhydrat-Antagonisten Endothelzellen zuzuführen, die CD34 exprimieren, worin der Antagonist an die Polypeptid-Hauptkette eines CD34-Polypeptids gebunden ist.

## Revendications

1. Polypeptide CD34 purifié, isolé ou un anticorps capable de lier CD34 natif pour une utilisation dans une méthode de traitement médical.

2. Utilisation d'un polypeptide CD34 purifié, isolé ou d'un anticorps capable de lier CD34 natif dans la préparation d'un médicament pour l'inhibition d'une condition pathologique associée à l'adhérence intercellulaire due à la L-sélectine.

3. Utilisation de la revendication 2 où la condition pathologique est associée à l'adhérence des leucocytes aux cellules endothéliales.

4. Utilisation de la revendication 3 où les leucocytes sont des lymphocytes et les cellules endothéliales sont sur les ganglions lymphatiques périphériques ou mésentériques.

5. Utilisation de la revendication 2 où la condition pathologique est une maladie autoimmune.

6. Utilisation de la revendication 5 où la condition pathologique est l'arthrite rhumatoïde, la sclérose multiple, le psoriasis, ou la dermatite chronique.

7. Utilisation de la revendication 3 où les leucocytes sont des neutrophiles ou des monocytes, et les cellules endothéliales sont celles de l'endothélium vénulaire.

8. Utilisation de la revendication 7 où la condition pathologique traitée est une inflammation aiguë ou chronique.

9. Utilisation de la revendication 7 où la condition pathologique est le syndrome de détresse respiratoire chez l'adulte (ARDS), une défaillance multi-organe, une blessure par reperfusion, une glomérulonéphrite aiguë, une arthrite réactive, une dermatose, une méningite purulente aiguë, une blessure thermique, une colite ulcérative, la maladie de Crohn, l'hémodialyse, la leucaphérèse, un choc hémorragique, ou une toxicité induite par les cytokines.

10. Utilisation de la revendication 3 comprenant de plus l'administration d'une quantité thérapeutiquement efficace d'un composé sélectionné dans le groupe consistant en :
(a) une sélectine;
(b) un ligand de sélectine autre qu'un polypeptide CD34;
(c) un anticorps capable de lier une sélectine ou un ligand de sélectine autre qu'un polypeptide CD34;
(d) une intégrine;
(e) un ligand d'intégrine;
(f) un anticorps capable de lier une intégrine ou un ligand d'intégrine; et
(g) un antagoniste non-protéine de l'interaction L-sélectine-CD34.

11. Utilisation de la revendication 10 où ledit composé est une P-sélectine, un ligand de P-sélectine ou un anticorps capable de lier la P-sélectine.

12. Utilisation selon l'une quelconque des revendications 2 à 11 comprenant de plus l'administration d'un agent antiinflammatoire stéroïde ou non stéroïde.

13. Utilisation selon l'une quelconque des revendications 2 à 12 où ledit patient est un mammifère, de préférence un humain.

14. Utilisation selon l'une quelconque des revendications 2 à 13 où ledit polypeptide CD34 a une structure de carbohydrate reconnue par l'anticorps monoclonal MECA 79.

15. Utilisation d'un anticorps capable de lier CD34 natif, où ledit anticorps est chimiquement ou physiquement associé à un composé pharmaceutiquement actif, dans la préparation d'un médicament pour le ciblage du composé pharmaceutiquement actif vers des cellules endothéliales.

16. Utilisation de la revendication 15 où ledit composé pharmaceutiquement actif est un agent antiinflammatoire ou un antioxydant.

17. Utilisation de la revendication 15 ou de la revendication 16 où ledit composé pharmaceutiquement actif est directement fusionné à une séquence de domaine constant dudit anticorps.

18. Molécule bispécifique comprenant une séquence de CD34 ou une séquence d'anticorps capable de lier CD34 natif et une autre fraction pharmaceutique active.

19. Molécule bispécifique de la revendication 18 comprenant une séquence d'anticorps capable de lier CD34 natif et une fraction pharmaceutiquement active d'un agent antiinflammatoire ou antioxydant.

20. Molécule bispécifique de la revendication 18 comprenant une première séquence d'anticorps capable de lier CD34 natif et une seconde séquence d'anticorps capable de lier une molécule différente associée à l'adhérence des leucocytes.

21. Molécule bispécifique de la revendication 20 où ladite seconde séquence d'anticorps est capable de lier un ligand de sélectine natif autre que CD34 ou est capable de lier un ligand d'intégrine natif.

22. Molécule bispécifique de la revendication 21 où ledit ligand d'intégrine est un membre de la famille de ICAM.

23. Composition pharmaceutique comprenant un anticorps anti-CD34.

24. Composition pharmaceutique de la revendication 23 comprenant de plus un composé pharmaceutiquement actif additionnel.

25. Utilisation d'un antagoniste carbohydrate de l'interaction L-sélectine-CD34 dans la préparation d'un médicament pour présenter ledit antagoniste carbohydrate aux cellules endothéliales exprimant CD34, ou ledit antagoniste est attaché à l'épine dorsale du polypeptide d'un polypeptide CD34.


### SEQUENCE LISTING

(1) GENERAL INFORMATION:
   (i) APPLICANT: Genentech, Inc.
      The Regents of the University of California
      Lasky, Laurence A.
      Baumhueter, Susanne
      Rosen, Steven D.
      Singer, Mark S.
   (ii) TITLE OF INVENTION: INHIBITION OF LEUKOCYTE ADHESION
   (iii) NUMBER OF SEQUENCES: 4
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 5.25 inch, 360 Kb floppy disk
      (B) COMPUTER: IBM PC compatible
      (C) OPERATING SYSTEM: PC-DOS/MS-DOS
      (D) SOFTWARE: patin (Genentech)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/056454
      (B) FILING DATE: 03-MAY-1993
   (viii) ATTORNEY/AGENT INFORMATION:
      (A) NAME: Dreger, Ginger R.
      (B) REGISTRATION NUMBER: 33,055
      (C) REFERENCE/DOCKET NUMBER: 833P1PCT
   (ix) TELECOMMUNICATION INFORMATION:
      (A) TELEPHONE: 415/225-3216
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 12 amino acids
      (B) TYPE: amino acid
      (D) TOPOLOGY: linear
   (xi) SEQUENCE DESCRIPTION: SEQ ID NO:1:
   Xaa Xaa Glu Thr Ser Xaa Gln Gly Ile Xaa Pro Thr
    1               5                   10      12
(2) INFORMATION FOR SEQ ID NO:2:
   (i) SEQUENCE CHARACTERISTICS:
      (A) LENGTH: 12 amino acids
      (B) TYPE: amino acid
      (D) TOPOLOGY: linear
   (xi) SEQUENCE DESCRIPTION: SEQ ID NO:2:
   Thr Thr Glu Thr Ser Thr Gln Gly Ile Ser Pro Ser
    1               5                   10      12
(2) INFORMATION FOR SEQ ID NO:3:
   (i) SEQUENCE CHARACTERISTICS:
      (A) LENGTH: 6 amino acids
      (B) TYPE: amino acid
      (D) TOPOLOGY: linear
   (xi) SEQUENCE DESCRIPTION: SEQ ID NO:3:
   Leu Gln Leu Met Glu Lys
    1               5   6
(2) INFORMATION FOR SEQ ID NO:4:
   (i) SEQUENCE CHARACTERISTICS:
      (A) LENGTH: 7 amino acids
      (B) TYPE: amino acid
      (D) TOPOLOGY: linear
   (xi) SEQUENCE DESCRIPTION: SEQ ID NO:4:
   Lys Leu Gln Leu Met Glu Lys
    1               5       7